(19) Europäisches Patentamt / European Patent Office / Office européen des brevets

(11) **EP 3 026 588 A1**

## (12) EUROPEAN PATENT APPLICATION

(43) Date of publication:
01.06.2016 Bulletin 2016/22

(51) Int Cl.:
*G06F 19/00* (2011.01)  *G06F 19/16* (2011.01)

(21) Application number: 14306882.3

(22) Date of filing: 25.11.2014

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME** | (72) Inventors:<br>• **Grudinin, Sergey**<br>  **38240 MEYLAN (FR)**<br>• **Derevyanko, Georgy**<br>  **38000 GRENOBLE (FR)**<br>• **Popov, Petr**<br>  **38240 MEYLAN (FR)**<br>• **Cheremovscky, Georgy**<br>  **38330 MONTBONNOT-SAINT-MARTIN (FR)** |
| (71) Applicants:<br>• **INRIA - Institut National de Recherche en Informatique et en Automatique**<br>  **78150 Le Chesnay (FR)**<br>• **Centre National de la Recherche Scientifique (C.N.R.S.)**<br>  **75016 Paris (FR)** | (74) Representative: **Cabinet Netter**<br>  **36, avenue Hoche**<br>  **75008 Paris (FR)** |

(54) **interaction parameters for the input set of molecular structures**

(57) The present invention relates to a method for modeling the geometric structure of the interface of Receptor-Ligand complexes, a method for modeling the interaction between a Receptor and a Ligand in Receptor-Ligand complexes, a method for determining a scoring vector w which is a mathematical vector quantifying and/or qualifying the interaction of a geometric structure of the interface of a Receptor-Ligand complex, a method for determining the binding affinity or binding free energy of a position of a Ligand relative to a Receptor in one or more Receptor-Ligand complexes, and a method for ranking the binding affinity or binding free energy of spatial positions of a Ligand relative to a Receptor in one or more Receptor-Ligand complexes.

Printed by Jouve, 75001 PARIS (FR)

**Description**

The present invention concerns a method for modeling the geometric structure of the interface of Receptor-Ligand complexes, a method for modeling the interaction between a Receptor and a Ligand in Receptor-Ligand complexes, a method for determining a scoring vector w which is a mathematical vector quantifying and/or qualifying the interaction of a geometric structure of the interface of a Receptor-Ligand complex, a method for determining the binding affinity or binding free energy of a position of a Ligand relative to a Receptor in one or more Receptor-Ligand complexes, and a method for ranking the binding affinity or binding free energy of spatial positions of a Ligand relative to a Receptor in one or more Receptor-Ligand complexes. All of these methods comprise one or more steps implemented or assisted by computer. The invention also relates to computer assisted design or representation of molecular structures, and more particularly of molecule interaction. The present invention also relates to any device implementing or helping to implement said methods, i.e. the corresponding software and hardware. The applications of the invention are all those where precise molecular interactions are important or crucial for the performance such as computer-aided drug design, pharmaceutical sciences, medicine, physics, and biology. Furthermore, the invention may present advantages for machine learning applications in computer graphics, computer vision, etc.

The average time required to develop a new active molecule, typically a drug, using the standard experimental analysis method Structure-Analysis-Relationship (SAR) is about 10-15 years with a cost of about $1.2 bin. Structure based drug design (SBDD) reduces drug design period to 7-12 years with a cost of about $1 bin, thus saving both time and money. There are thus huge needs to decrease either the duration to develop a new active molecule, the cost thereof, or even preferably both.

In particular, the invention provides a way to perform fast, accurate and efficient virtual screening of potential drug molecules, which is the initial step of the drug design pipeline.

Given a complex of interacting molecules, it is technically difficult to quantify the interaction between the interacting molecules, ideally corresponding to the experimental values of the binding free energy or the binding affinity. It is thus very difficult to predict the binding affinity of a receptor-ligand complex, and thereby difficult to rank and select the best (minimal) binding free energy or the binding affinity in order to select the best candidate(s) for drug design. There is a need to overcome such technical problems.

Despite the vast variety of the methods to obtain the scoring functions (SFs), they can be clustered into three major classes: forcefield-based SFs, empirical SFs and statistical SFs.

- Forcefield-based (FF) SFs present the score as a decomposition of the free energy into individual physics-based interaction terms such as van der Waals, electrostatics, bond stretching, bending, etc. energies. Classical molecular mechanical force fields such as AMBER or CHARMM are widely used for this purpose. Major challenges of the Forcefield-based SFs are: 1) accounting for the solvent molecules; 2) accounting for entropic effect; 3) and the possibility of decomposing the binding free energy into a linear combination of interaction terms. GOLD::GoldScore and SYBYL::G-Score/D-Score are the forcefield-based SFs evaluated by Cheng et al[1]. FF scoring functions are also used in DOCK and AutoDock packages. Overall, FF scoring functions have a rather poor performance[1] and there is no rigorous way to adjust weights between different interaction terms.
- Empirical SFs are constructed as a weighted sum of terms, such as desolvation, electrostatic interactions, hydrogen bonds, hydrophobic interactions, etc., $\alpha_i$: $E = \sum_i \alpha_i E_i$. Then, coefficients $\alpha_i$ are tuned to match some experimental data, such as binding affinity, or to attain a minimum of the scoring function on the known native structures. Regression analysis is typically used for these purposes. Empirical scoring functions are much more computationally efficient in comparison with the FF scoring function[2]: Glide, ICM, LUDI, PLP, ChemScore, X-Score, Surflex, SYBYL/F-Score, MedusaScore, AIScore, SFCscore are some examples of the empirical-based scoring functions. Overall, Empirical SFs perform better compared to FF scoring functions[1] but posses the same problem of adjusting the weights between their interaction terms.
- Statistical scoring functions, on the other hand, are based on the observation that the distances between the atoms in experimentally determined structures follow the Boltzmann distribution. More precisely, using ideas from statistical theory of liquids, effective potentials between atoms are extracted using the inverse Boltzmann relation: $E_{ij}(r) = -k_B T \ln(P_{ij}(r)/Z)$, where $k_B T$ is the Boltzmann constant, $P_{ij}(r)$ denotes the probability to find two atoms of types $i$ and $j$ at a distance r, and Z denotes the probability distribution in the reference state. The latter is the thermodynamic equilibrium state of the protein when all interactions between the atoms are set to zero. The score of a protein conformation is then given as a sum of effective potentials between all pairs of atoms. Although this concept is old (it originates from the work of Tanaka and Scheraga[3], Miyazawa and Jernigan[4], and Sippl[5]), it is still under debates. Particularly, the computation of the reference state is a challenging problem and only recently some attempts to rigorously justify and compute it have been made. ITScore, PMF, DrugScore, DFIRE, BLEEP, MScore, GOLD/ASP are some knowledge-based scoring functions. GOLD::ASP, DS::PMF, SYBYL::PMF, and DrugScore were evaluated in Cheng et al comparative assessment[1]. Overall, Statistical SFs are the winners in all types of benchmarks and

competitions[1], however they typically have thousands of parameters, which are extremely sensitive to the training sets of molecular structures and parameters of the optimization algorithms.

Despite these improvements, there is still a need to discover a new way to perform faster, more accurate and more efficient virtual screening of potential drug molecules.

The invention thus aims to solve the above-described problems.

More precisely, the methods of the invention and the associated algorithms are very fast, robust, general, and stable to noise in initial structures, as verified on a number of different benchmarks. Therefore the present invention represents an important improvement for modeling of geometric structure of an interface of Receptor-Ligand complexes, for modeling an interaction between a Receptor and a Ligand in Receptor-Ligand complexes, for determining a scoring vector quantifying and/or qualifying the interaction of a geometric structure of an interface of a Receptor-Ligand complex, for determining the binding affinity or binding free energy of a position of a Ligand relative to a Receptor in one or more Receptor-Ligand complexes, and for ranking the binding affinity or binding free energy of spatial positions of a Ligand relative to a Receptor in one or more Receptor-Ligand complexes.

The invention is described below in more details.

The invention relates to a method for modeling the geometric structure of the interface of Receptor-Ligand complexes, wherein a first chemical molecule defined as Receptor and a second chemical molecule defined as Ligand, wherein said method comprises the following steps wherein at least one of them is implemented or assisted by computer:

(a) providing a set of Receptors and Ligands from at least one computer database, wherein Receptor-Ligand complexes present an interface comprising different atom types, wherein atom type k is located on the Receptor and atom type I is located on the Ligand interact, k and I varying depending on the atom type;

(b) selecting atoms from at the Receptor-Ligand complexes interface of said Receptors and Ligands;

(c) assigning to each selected atoms an atom type among k and I;

(d) providing for Receptor-Ligand complexes the distances $r_{ij}$ between an atom i of a specific atom type k of the Receptor and an atom j of a specific atom type I of the Ligand, wherein i index runs over specific atoms among atom type k, and wherein j index runs over specific atoms among atom type I ;

(e) optionally repeating step (c) for all or other atom types k and I;

(f) assigning the distances $r_{ij}$ as a function of atom types; and

(g) providing the modeling of the geometric structure of the interface of Receptor-Ligand complexes as a function of distances $r_{ij}$.

In one embodiment, the interface is a set of all atom pairs ij at a distance smaller than the cutoff distance $r_{max}$ such that the first atom i in each pair ij belongs to the receptor and the second atom j in each pair ij belongs to the ligand.

For example the interface is a set of atoms determined using the standard linked-cell algorithm. More precisely, using a grid initialized with atoms of the receptor, atoms of the receptor-ligand interface are selected, in linear time, as those wherein distance $r_{ij}$ is less than the cutoff distance.

In one embodiment, Atom types are defined by the classification of all heavy atoms (that is all atoms excluding hydrogens) according to their element symbol, aromaticity, hybridization, and polarity. Sybyl atom types can be used, for example[6]. In this case, the atom types can be computed by Sybyl, OpenBabel or other widely-used molecular software such as DOCK. Alternatively, manual conversion tables are provided in the literature, for example, in the RPluto user guide from the CSD System package.

Receptors and ligands can be represented as a set of discrete interaction sites located at the centers of the atomic nuclei, thereby forming the interacting interface.

All atoms may be divided for example into M atom types according to the properties of corresponding atomic nuclei (element type, charge, hydrophobicity, etc.). Thereby, each atom has the associated position and atom type. Such atoms may also be defined as interaction sites.

These result in total in *Mx (M + 1)/2* pairs of atom types.

Atom types were assigned to the atoms for example according to their surrounding and functional groups they consist in. To do so, it can for example be used the fconv library[7] for atom typization, which provides 158 internal atom types. Then, the atom types are clustered into 48 groups by measuring the statistical similarity of pair-distribution functions between different atom types in the training data set. Atom types set used to describe proteins and ligands can be the same, despite the fact that proteins always contain atoms of only some specific types. In one embodiment, the parameterization consists of 48 atom types. More precisely, such atom types are: 17 types for nitrogen, 9 types for oxygen, 8 types for carbon, 4 types for sulfur, 2 types for phosphorus and 8 types for halogens.

Preferably, said geometric structure is defined as a structure vector x comprising as coordinates distances $r_{ij}$ as a function of atom types.

In one embodiment, said structure vector x depends on various atom types in the Receptor-Ligand complex and on the distance between various atom types in the Receptor-Ligand complex. This may be obtained according to equation (11):

$$x_p^{kl} = \int_{r_1}^{r_2} n_{kl}(r)\xi_p(r)\sqrt{\Omega(r)}\,dr \qquad\qquad (1)$$

In one embodiment, said modeling of the geometric structure of the interface of Receptor-Ligand complexes takes into account inaccuracies in the determination of distances $r_{ij}$. In one embodiment, inaccuracies in the distance $r_{ij}$ are taken into account in the method of the invention.

In one embodiment, the modeling of the geometric structure of the interface of Receptor-Ligand complexes as a function of distances $r_{ij}$. is defined by the number densities $n^{kl}(r)$, wherein said number densities $n^{kl}(r)$ is defined as:

$$n^{kl}(r) = \sum_{ij} \frac{1}{\sqrt{2\pi\sigma^2}} e^{-\frac{(r-r_{ij})^2}{2\sigma^2}}, \qquad (4)$$

wherein each distance distribution is represented by a Gaussian centered at $r_{ij}$ with the constant variance of $\sigma^2$, and wherein the distance $r_{ij}$ is smaller than a determined cutoff distance $r_{max}$.

In one embodiment, said linear scoring function F is defined by equation (1):

$$F\big(n(r)\big) \equiv F\big(n^{11}(r),..,n^{kl}(r),..,n^{MM}(r)\big) = \sum_{k=1}^{M} \sum_{l=k}^{M} \int_{0}^{r_{max}} n^{kl}(r)U^{kl}(r)\,dr \qquad\qquad (2)$$

wherein unknown "scoring potentials" functions $U^{kl}(r)$ can be determined from a training set of native complexes.

Advantageously, the cutoff distance is set between 1 and 20, preferably between 6 and 12 Angstrom (A). Advantageously, the cutoff distance is 10 Angstrom (A).

In one embodiment, the value of $\sigma$ is assumed to be equal for all types of site-site interactions and determined from the cross-validation procedure.

In one embodiment, additional information is used for more precise parameterization of variance or even instead of the Gaussian approximation in Eq. (4). Such additional information is for example: individual distance distributions, e.g. Debye-Waller factors, molecular dynamics trajectories, etc.

The invention also relates to a method of generating virtual Non-Native Receptor-Ligand complexes, wherein said method comprises the following steps wherein at least one of them is implemented or assisted by computer:

(a) providing a set of Native Receptor-Ligand complexes $P_i^{nat}$ from at least one computer database, wherein Receptor-Ligand complexes present an interface wherein site k of the Receptor and site I of the Ligand interact;

(b) generating D Non-Native Receptor-Ligand complexes $P_j^{nonnat}$, ,j = 1... D wherein j index runs over generated decoys and D represents the total number of Non-Native Receptor-Ligand complexes generated, wherein Non-Native Receptor-Ligand complexes are generated by moving spatially the Ligand relative to the Receptor or by local deformation along spatial directions from the Native Receptor-Ligand complexes.

In one embodiment, Non-Native Receptor-Ligand complexes $P_j^{nonnat}$, are generated by rolling the Ligand over the surface of the Receptor. For example, this can be performed using the Hex algorithm.

In one embodiment, Non-Native Receptor-Ligand complexes $P_j^{nonnat}$, are generated by the following steps:

- Considering a Ligand as a rigid body,
- Rotating the Ligand about one or more rotational axes, and
- Translating the Ligand along the coordinate axes.

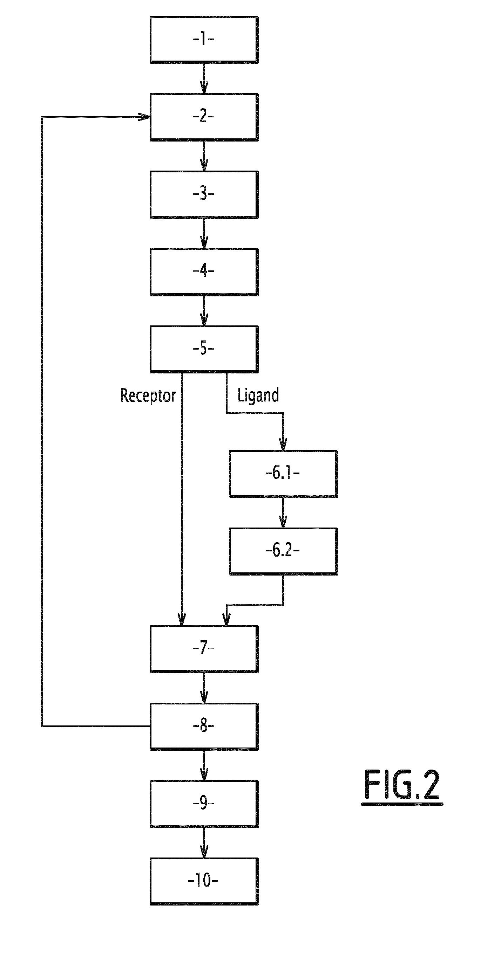

By reference to figure 2, it is preferred for small molecules or chemical drug that corresponding decoy is generated by setting axes, for example 6 axes, inside a unit sphere corresponding to its icosahedral tessellation; then by rotating the ligand about these axes such that RMSD is kept constant, and then by setting six translations along the coordinate axes; and translating the ligand by RMSD amount. Small molecules are defined as molecule presenting a molecular weight below 900 Daltons. Small molecules have a size in general of less than $10^{-9}$ m.

In one embodiment, Non-Native Receptor-Ligand complexes $P_j^{nonnat}$, are generated by linear combinations of modes $\{v_i\}$ as follows:

$$\mathbf{X}^{decoy} = \mathbf{X}^{native} + \sqrt{k_B \mathrm{T}} \sum_{i=6}^{15} r_i \frac{v_i}{\omega_i},$$

where $\mathbf{X}^{native}$ and $\mathbf{X}^{decoy}$ are the coordinate vectors corresponding to the native and non-native conformations, respectively, $r_i$ is the random weight for each mode ranging from -1 to 1, and $\omega_i$ is the frequency of the mode $v_i$.

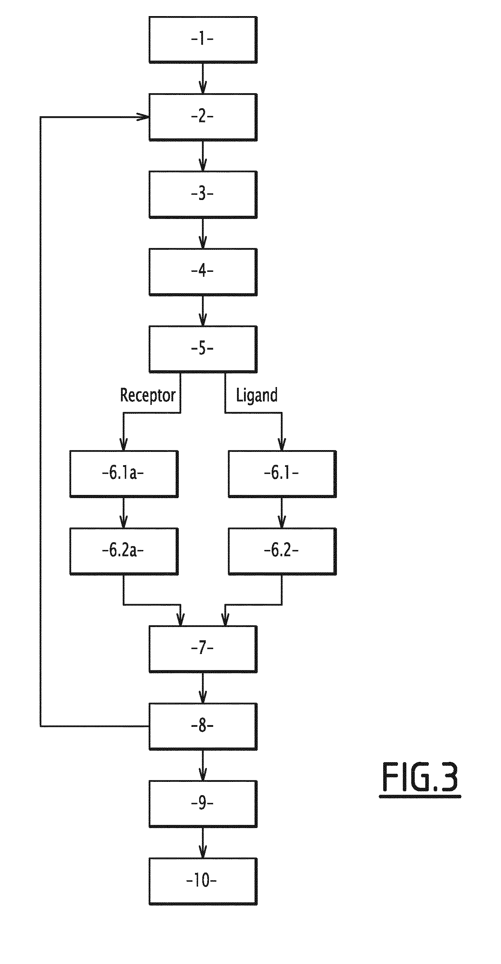

By reference to figure 3, it is preferred for Protein-Protein interaction to contract the Hessian Matrix and compute its eigenvectors $L_i$ (for example the 10 first eigenvectors) and then generate decoys (for example 15).

In one embodiment, the modes are obtained by the diagonalization of the Hessian matrix H, which is the matrix of second derivatives of, for example, the OPLS potential function with respect to atomic positions, as $H = V \Lambda V^T$. Here, V is a unitary matrix, composed of the eigenvectors $v_i$ of H and $\Lambda$ is the diagonal matrix of eigenvalues $\lambda_i$. The frequency and shape of a mode is represented by its eigenvalue and eigenvector, respectively. The frequency of a mode $\omega_i$ is given as the square root of the corresponding eigenvalue, $\omega_i = \sqrt{\lambda_i}$.

In one embodiment, rolling the Ligand over the surface of the Receptor is performed by the Hex protein docking software[8]

In one embodiment, Receptor-Ligand complex is labeled as "native" if the root mean square deviation (RMSD) of the corresponding Ligand is less than a determined value, for example < 2A, from its native position. Otherwise, the Receptor-Ligand complex is labeled as "non-native" or "decoy".

The invention also relates to a method for modeling the interaction between a Receptor and a Ligand in Receptor-Ligand complexes,
wherein said method comprises the following steps wherein at least one of them is implemented or assisted by computer:

(a) providing a set of Receptors and Ligands from at least one computer database;
(b) assigning to each Receptor-Ligand complex a specific structure vector x which is a mathematical vector representing the specific geometric structure of the interface between a Receptor and a Ligand in a specific Receptor-Ligand complex;
(c) computing a linear convex scoring function F as a function of all specific structure vectors x or of vector X which is the concatenation of all vectors x thereof, preferably said linear convex scoring function F being also a function of a scoring vector w;
(d) projecting said scoring function F in orthogonal polynomial subspaces; thereby modeling the interaction between a Receptor and a Ligand in Receptor-Ligand complexes.

According to one embodiment, said method comprises:

(a1) providing a set of Receptor-Ligand complex configuration $P_i^{nat}$, $i = 1 ... N$, from at least one computer database,
wherein $P^{nat}$ is a native Receptor-Ligand complex configuration and i is an integer assigned to a Receptor-Ligand complex configuration
(a2) calculating a scoring functional F for different Receptor-Ligand complexes wherein for each native complex i and its non-native decoy j the following inequality holds:

$$F\left(P_i^{nat}\right) < F\left(P_{ij}^{nonnat}\right) \qquad\qquad (3)$$

wherein F is a linear convex function depending on the geometrical interface between the Receptor and the Ligand,
wherein Receptors and Ligands are represented as a set of discrete interaction at the interface of the Receptor-Ligand complex(es), and
wherein the interface is a set of all atom pairs at a distance smaller than the cutoff distance $r_{max}$ such that the first atom in each pair belongs to the Receptor and the second atom in each pair belongs to the Ligand.

In one embodiment, F is represented as a function of the distribution of the distances between the atoms of the interface, represented by (3):

$$F(P) \equiv F\left(n^{11}(r),..,n^{kl}(r),..,n^{mm}(r)\right) \equiv F\left(n(r)\right) \qquad (4)$$

wherein $n^{kl}(r)$ is the number density of atom-atom at a distance r between two atom types k and l, with atom type k on the Receptor, and atom type l on the Ligand, where m is the total number of different atoms in a Receptor-Ligand complex interface.

The invention also relates to a method for determining a scoring vector **w** which is a mathematical vector quantifying and/or qualifying the interaction of a geometric structure of the interface of a Receptor-Ligand complex, wherein Receptor-Ligand complexes present an interface in interaction, wherein said interaction is in need for quantification and/or qualification, wherein said method comprises the following steps wherein at least one of them is implemented or assisted by computer:

(a) providing a set of Receptors and Ligands from at least one computer database;
(b) assigning to each geometric structure of an interface of a Receptor-Ligand complex, a specific structure vector **x** which is a mathematical vector representing the specific geometric structure of the interface;
(c) computing a linear convex scoring function F as a function of all specific structure vectors x and scoring vector **w**;
(e) projecting said scoring function F in orthogonal polynomial subspaces;
(f) formulating a convex optimization problem;
(g) solving the convex optimization problem thereby determining a scoring vector **w**.

In one embodiment, said method comprises computing the score of each conformation using the following equation (4):

$$\text{Score} = \sum_{ij} \Upsilon^{kl}(r_{ij}) \qquad (5)$$

wherein the sum is taken over all pairs of atoms i and j separated by the distance $r_{ij}$ smaller than the threshold $r_{max}$, with atom i of type k on the Receptor, and atom j of type l on the Ligand, and

In one embodiment, functions $\gamma^{kl}(r)$ are computed as a convolution $\gamma^{kl} = f * U^{kl}$.

In one embodiment, functions $\gamma^{kl}(r)$ are the Gauss transform (5) of the scoring potential $U^{kl}(x)$:

$$\Upsilon^{kl}(r) = \frac{1}{\sqrt{2\pi\sigma^2}} \int_0^{r_{max}} e^{-\frac{(x-r)^2}{2\sigma^2}} U^{kl}(x) dx \qquad (6)$$

In one embodiment, said scoring function F is projected in orthogonal polynomial subspaces.

In a particular embodiment, the polynomial expansion of the scoring functional F(n(r)) is defined by (6):

$$F(n(r)) = \sum_{k,l}^{M} \int_0^{r_{max}} \sum_{p_1} \sum_{p_2} w_{p_1}^{kl} x_{p_2}^{kl} \xi_{p_1}(r) \xi_{p_2}(r) \Omega(r) dr \qquad (7)$$

wherein functions $\xi_p(r)$ are orthogonal on the interval $[r_1; r_2]$ with a nonnegative weight function $\Omega(r)$ defined by (7):

$$\int_{r_1}^{r_2} \xi_{p_1}(r) \xi_{p_2}(r) \Omega(r) dr = \delta_{p_1 p_2}, \qquad (8)$$

wherein $\delta_{pip2}$ is the Kronecker delta function, the scoring potentials $U_{kl}(r)$ and number densities $n_{kl}(r)$ can be expanded on the interval $[r_1; r_2]$ as

$$U_{kl}(r) = \sum_p w_p^{kl} \xi_p(r) \sqrt{\Omega(r)}, r \in [r_1; r_2] \qquad (9)$$

$$n_{kl}(r) = \sum_p x_p^{kl} \xi_p(r) \sqrt{\Omega(r)}, r \in [r_1; r_2] \qquad (10)$$

wherein expansion coefficients $w_p^{kl}$ and $x_p^{kl}$ can be determined from the orthogonality condition (7) as

$$w_p^{kl} = \int_{r_1}^{r_2} U_{kl}(r) \xi_p(r) \sqrt{\Omega(r)} dr \qquad (11)$$

$$x_p^{kl} = \int_{r_1}^{r_2} n_{kl}(r) \xi_p(r) \sqrt{\Omega(r)} dr \qquad (12)$$

In one embodiment, said polynomial expansion of the scoring functional F is defined by

$$F\big(n(r)\big) \approx \sum_{k=1}^{M} \sum_{l=k}^{M} \sum_p^P w_p^{kl} x_p^{kl} = (\mathbf{w} \cdot \mathbf{x}), \mathbf{w}, \mathbf{x} \in \mathbb{R}^{P \times M \times (M+1)/2} \quad (13):$$

$$F\big(n(r)\big) \approx \sum_{k=1}^{M} \sum_{l=k}^{M} \sum_p^P w_p^{kl} x_p^{kl} = (\mathbf{w} \cdot \mathbf{x}), \mathbf{w}, \mathbf{x} \in \mathbb{R}^{P \times M \times (M+1)/2} \qquad (13)$$

wherein the vector w is the scoring vector and the vector x is the structure vector.

wherein $\mathbb{R}^{l\ P \times M \times (M+1)/2}$ is a scoring space of a projection from a Receptor-Ligand complex.

In one embodiment, a convex optimization problem is formulated from polynomial expansion of the scoring functional F defined by (12).

More particularly, a smooth convex optimization problem is formulated or a kernelized smooth optimization problem is formulated.

The invention also relates to a method for finding an optimal scoring vector for modeling Receptor-Ligand complex configurations wherein said scoring vector is defined as scoring vector **w**,

wherein $\mathbf{w} \in \mathbb{R}^{P \times M \times (M+1)/2}$

and wherein

$$\forall i = 1 \dots N, \ \forall j = 1 \dots D \quad (\mathbf{x}_i^{nat} \cdot \mathbf{w}) < (\mathbf{x}_{ij}^{nonnat} \cdot \mathbf{w}), \qquad (14)$$

or, equivalently,

$$\forall i = 1 \dots N, \ \forall j = 1 \dots D \quad ([\mathbf{x}_{ij}^{nonnat} - \mathbf{x}_i^{nat}] \cdot \mathbf{w}) > 0), \qquad (15)$$

which defines a set of $N \times D$ *half-space equations* in $\mathbb{R}^{P \times M \times (M+1)/2}$ with *N* parallel separation hyperplanes defined by the normal w.

In such embodiment, finding the scoring vector is equivalent to finding the common normal to the *N* planes defined by Eq. (14).

In one embodiment, the solution is given by minimizing a smooth and convex function defined by (15):

$$\text{Minimize(in } \mathbf{w}, b_j) \frac{1}{2}\mathbf{w} \cdot \mathbf{w} + \text{Loss}(\mathbf{w}, \mathbf{x}, \mathbf{b}) \qquad\qquad (\,16\,)$$

Wherein Loss is a loss function depending on **w, x** and **b,**
wherein **w** is the scoring vector and the vector x is the structure vector defined by (10-11).

Wherein $b_j$ are the offset parameters, which determine the offset of the hyperplanes from the origin along the scoring vector **w.**

In one embodiment, the scoring vector **w** is a linear combination of the support vectors.

In one embodiment, the invention uses kernelized version of the Smooth Convex Optimization Problem.

In one embodiment, said step (a) comprises providing Native Receptor-Ligand complex $P_i^{nat}$ and Non-native Receptor-Ligand complexes $P_{ij}^{nonnat}$, wherein *i* index runs over different protein complexes.

In one embodiment, said step (b) comprises implementing a method for modeling the geometric structure of the interface of Receptor-Ligand complexes as defined in the present invention.

In one embodiment, in step (e) orthogonal polynomial subspaces are Rectangular, Legendre, Laguerre or Fourier orthogonal bases.

In one embodiment, step (f) comprises using the artificially generated noise applied to the original input data.

In one embodiment, said noise is represented by the Gaussian distance distribution of the input data having a variance $\sigma$ where $\sigma$ is constant and does not depend on the atom type. This noise can be thought as a Gaussian filter applied to the input data if the latter is represented as a 1 D signal.

In one embodiment, step (f) comprises formulating a convex optimization problem so as to minimize the convex function.

In one embodiment, step g) (solving the convex optimization problem thereby determining a scoring vector w) comprises implementing at least one solver selected from the group consisting of the coordinate-descent solver, Nesterov descent solver, a stochastic gradient solver, the quasi-Newton family solvers (e.g. BFGS), and any combination thereof.

Preferably, said method further comprises finding the scoring vector **w.**

The invention also relates to a method for determining the binding affinity or binding free energy of a position of a Ligand relative to a Receptor in one or more Receptor-Ligand complexes, wherein said Receptor-Ligand complex present an interface comprising different atom types, wherein atom type k, located on the Receptor, and atom type l, located on the Ligand, interact, k and l varying depending on the atom type, wherein said method comprises the following steps wherein at least one of them is implemented or assisted by computer:

(i) modeling the geometric structure of the interface of one or more Receptor-Ligand complexes, said modeling being as defined in the present invention;

(ii) assigning to a geometric structure of the interface of Receptor-Ligand complex, a binding affinity or binding free energy by reference to a database, optionally wherein said binding affinity or binding free energy is determined as a scalar product of the structure vector *x* with the scoring vector **w** as defined in the method for determining a scoring vector **w** as defined in the present invention.

The invention also relates to a method for ranking the binding affinity or binding free energy of spatial positions of a Ligand relative to a Receptor in one or more Receptor-Ligand complexes, wherein said method comprises the following steps wherein at least one of them is implemented or assisted by computer:

(i) implementing the method for determining the binding affinity or binding free energy of a position of a Ligand relative to a Receptor in one or more Receptor-Ligand complexes, to determine the binding affinity or binding free energy of two or more spatial positions of a Ligand relative to a Receptor in one or more Receptor-Ligand complexes,

(ii) ranking spatial positions of a Ligand relative to a Receptor based on said binding affinity or binding free energy by providing a strict relationship between a set of spatial positions such that, for any two positions, the first is either ranked higher, ranked lower or ranked equal to the second position if the binding energy of the first position is smaller, equal or higher than the energy of the second position, respectively.

Advantageously, the best spatial position of a Ligand relative to a Receptor in one or more Receptor-Ligand complexes is determined based on the ranking of said binding affinity or binding free energy.

Advantageously, the best binding affinity or free binding energy among several Receptor-Ligand complexes is determined based on the ranking of said binding affinity or binding free energy.

In the past years, a number of methods to derive scoring functions of different types and properties have been proposed as it is described above. Input information for these methods (structural data, statistical data) is taken from the experiments (X-Ray crystallography, NMR, binding affinity measurements, etc.). However, experimental data is always biased towards certain experimental conditions and always contains standard implementation errors of different types. One of the main advantageous distinction of the invention comprises the accounting for experimental error by introducing uncertainties during the training process (as implemented in the statistical kernel). Different type of kernels might be used, for example, the invention may use the Gaussian kernel, which allows to deal with uncertainties in the experimental data by representing these data as a "dome" centered at the exact experimental measures (for example Eq. (23)). Thus, the structure vectors according to the invention built upon the kerneled experimental data are much more robust, meaning that they represent the real, unbiased, data more accurately without statistical bias. As a consequence, the derived scoring function is also robust and steady to the experimental biases, providing better performance compared to the state-of-the-art scoring functions as it is demonstrated in the example below.

The invention also relates to a software for modeling the geometric structure of the interface of Receptor-Ligand complexes, wherein the software is embodied in a computer readable media and when executed said software implements the method for modeling the geometric structure of the interface of Receptor-Ligand complexes according to the invention.

The invention also relates to a software for generating virtual Non-Native Receptor-Ligand complexes, wherein the software is embodied in a computer readable media and when executed said software implements the method for generating virtual Non-Native Receptor-Ligand complexes according to the invention.

The invention also relates to a software for modeling the interaction between a Receptor and a Ligand in Receptor-Ligand complexes, wherein the software is embodied in a computer readable media and when executed said software implements the method for modeling the interaction between a Receptor and a Ligand in Receptor-Ligand complexes according to the invention.

The invention also relates to a software for determining a scoring vector *w* which is a mathematical vector quantifying and/or qualifying the interaction of a geometric structure of the interface of a Receptor-Ligand complex, wherein the software is embodied in a computer readable media and when executed said software implements the method for determining a scoring vector w which is a mathematical vector quantifying and/or qualifying the interaction of a geometric structure of the interface of a Receptor-Ligand complex according to the invention.

The invention also relates to a software for determining the binding affinity or binding free energy of a position of a Ligand relative to a Receptor in one or more Receptor-Ligand complexes, wherein the software is embodied in a computer readable media and when executed said software implements the method for determining the binding affinity or binding free energy of a position of a Ligand relative to a Receptor in one or more Receptor-Ligand complexes according to the invention.

The invention also relates to a software for ranking the binding affinity or binding free energy of spatial positions of a Ligand relative to a Receptor in one or more Receptor-Ligand complexes, wherein the software is embodied in a computer readable media and when executed said software implements the method for ranking the binding affinity or binding free energy of spatial positions of a Ligand relative to a Receptor in one or more Receptor-Ligand complexes according to the invention.

The invention also relates to a hardware comprising at least one software as described in the present description.

The invention also relates to a system for generating virtual Non-Native Receptor-Ligand complexes, said system comprising means for generating virtual Non-Native Receptor-Ligand complexes according to the invention.

The invention also relates to a system for modeling the geometric structure of the interface of Receptor-Ligand complexes, said system comprising:

(a) means for providing a set of Receptors and Ligands from at least one computer database, wherein Receptor-Ligand complexes present an interface comprising different atom types, wherein atom type k is located on the Receptor and atom type l is located on the Ligand, k and l varying depending on the atom type;
(b) means for selecting atoms from at the Receptor-Ligand complexes interface of said Receptors and Ligands;
(c) means for assigning to each selected atoms an atom type among k and l;
(d) means for providing for Receptor-Ligand complexes the distances $r_{ij}$ between an atom i of a specific atom type k of the Receptor and an atom j of a specific atom type l of the Ligand, wherein i index runs over specific atoms among atom type k, and wherein j index runs over specific atoms among atom type l ;
(e) optionally means for repeating step (c) for all or other atom types k and l;
(f) means for assigning the distances $r_{ij}$ as a function of atom types; and
(g) means for providing the modeling of the geometric structure of the interface of Receptor-Ligand complexes as a function of distances $r_{ij}$, preferably said geometric

structure is defined as a structure vector x comprising as polynomial coefficients of coordinates distances $r_{ij}$ as a function of atom types computed in an orthogonal polynomial basis.

The invention also relates to a system for determining a scoring vector **w** which is a mathematical vector quantifying and/or qualifying the interaction of a geometric structure of the interface of a Receptor-Ligand complex, said system comprising:

(a) means for providing a set of Native Receptor-Ligand complexes $P_i^{nat}$ from at least one computer database, wherein Receptor-Ligand complexes present an interface wherein site k of the Receptor and site I of the Ligand interact; and

(b) means for generating D Non-Native Receptor-Ligand complexes $P_j^{nonnat}$, $j$ = 1... D wherein $j$ index runs over generated decoys and D represents the total number of Non-Native Receptor-Ligand complexes generated, wherein Non-Native Receptor-Ligand complexes are generated by moving spatially the Ligand relative to the Receptor or by local deformation along spatial directions from the Native Receptor-Ligand complexes.

The invention also relates to a system for modeling the interaction between a Receptor and a Ligand in Receptor-Ligand complexes, wherein said system comprises:

(a) means for providing a set of Receptors and Ligands from at least one computer database;
(b) means for assigning to each Receptor-Ligand complex a specific structure vector x which is a mathematical vector representing the specific geometric structure of the interface between a Receptor and a Ligand in a specific Receptor-Ligand complex;
(c) means for computing a linear convex scoring function F as a function of all specific structure vectors x or of vector X which is the concatenation of all vectors x thereof, preferably said linear convex scoring function F being also a function of a scoring vector w ; and
(d) means for projecting said scoring function F in orthogonal polynomial subspaces; thereby modeling the interaction between a Receptor and a Ligand in Receptor-Ligand complexes.

The invention also relates to a system for determining a scoring vector w which is a mathematical vector quantifying and/or qualifying the interaction of a geometric structure of the interface of a Receptor-Ligand complex, wherein said system comprises:

(a) means for providing a set of Receptors and Ligands from at least one computer database;
(b) means for assigning to each geometric structure of an interface of a Receptor-Ligand complex, a specific structure vector **x** which is a mathematical vector representing the specific geometric structure of the interface;
(c) means for computing a linear convex scoring function F as a function of all specific structure vectors **x** and scoring vector **w**;
(e) means for projecting said scoring function F in orthogonal polynomial subspaces;
(f) means for formulating a convex optimization problem; and
(g) means for solving the convex optimization problem thereby determining a scoring vector **w.**

The invention also relates to a system for determining the binding affinity or binding free energy of a position of a Ligand relative to a Receptor in one or more Receptor-Ligand complexes, wherein said system comprises:

(i) means for modeling the geometric structure of the interface of one or more Receptor-Ligand complexes, said modeling being as defined according to the present invention;
(ii) means for assigning to a geometric structure of the interface of Receptor-Ligand complex, a binding affinity or binding free energy by reference to a database, optionally wherein said binding affinity or binding free energy is determined using a scoring vector w as defined in the method for determining a scoring vector w which is a mathematical vector quantifying and/or qualifying the interaction of a geometric structure of the interface of a Receptor-Ligand complex.

The invention also relates to a system for ranking the binding affinity or binding free energy of spatial positions of a Ligand relative to a Receptor in one or more Receptor-Ligand complexes, wherein said system comprises:

(i) means for implementing the method of determining the binding affinity or binding free energy of a position of a Ligand relative to a Receptor in one or more Receptor-Ligand complexes according to the present invention to determine the binding affinity or binding free energy of two or more spatial positions of a Ligand relative to a Receptor in one or more Receptor-Ligand complexes, and

(ii) means for ranking a set of positions of a Ligand relative to a Receptor by providing a strict relationship between the set such that, for any two positions, the first is either ranked higher, lower or equal to the second position if the said binding free energy of the first position is smaller, equal or higher than the energy of the second position, respectively in one or more Receptor-Ligand complexes according to the present invention to determine the top binding poses of a Ligand relative to a Receptor in one or more Receptor-Ligand complexes.

The invention also relates to the corresponding software and parameter datasets.

The invention also relates to a method for predicting molecule-molecule interactions, for example protein-protein, protein-drug, in particular protein-small molecule interaction, wherein said method comprises implementing at least one method as defined according to the invention.

The invention also relates to a method for designing molecules, for example drugs, proteins, peptides, polypeptides, or other small molecules, wherein said method comprises implementing at least one method according to the present invention.

The present invention has also applications where precise molecular interactions are crucial for the performance such as computer-aided drug design, pharmaceutical sciences, medicine, physics, and biology. Furthermore, the invention relates also to machine learning applications for example in in computer graphics, computer vision, etc.

Currently, the invention provides a solution for companies, especially pharmaceutical companies and organisations working in biological and medical research in general. In particular, the invention provides a way to perform fast, accurate and efficient virtual screening of potential drug molecules, which is the initial step of the drug design pipeline.

The method according to the invention is very fast and general with respect to classes of input molecules.

In the Figures:

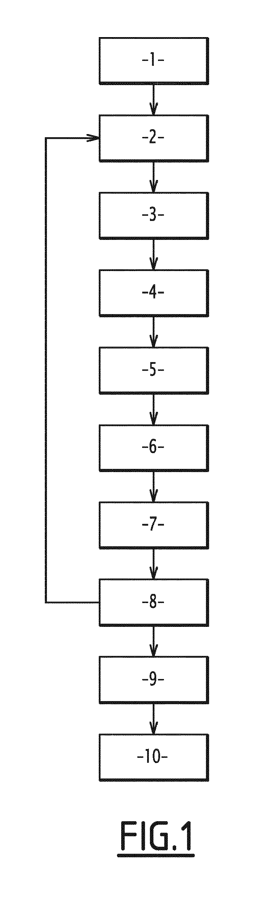

**Figure 1** represents a flowchart of main steps of a method according to the present invention, said method comprising the following steps:

(1) loading of i=1... N structures of native complexes,
(2) performing the following steps (3) to (7) for each complex i:
(3) assigning to each atom the associated atom type;
(4) find all pairs of ligand-receptor atoms separated by less than ten Angstroms,

(5) computing one native structure vector $X_i^{nat}$;

(6) generating decoys;
(7) constructing near-native conformations: for each conformation find all pairs of ligand-receptor atoms separated by less than ten Angstroms;
then,

(8) computing non-native structure vectors $X_{ij}^{nonnat}$;

(9) formulating the optimization problem, given native and non-native structure vectors; and
(10) solving the optimization problem for scoring vectors **w.**

**Figure 2** is a flowchart representing a method which includes generating decoys for protein-small drug interaction, wherein step (6) of figure 1 is further detailed and comprises the following steps for the Ligand (small drug):

(6.1) setting six axes inside a unit sphere corresponding to its icosahedral tessellation, and (6.2) rotating the ligand about these axes such that RMSD is kept constant, and setting six translations along the coordinate axes; and translating the ligand by RMSD amount.

**Figure 3** is a flowchart representing a method which includes generating decoys for protein-protein interaction, wherein step (6) of figure 1 is further detailed and comprises the following steps:

For the Ligand: (6.1) constructing the Hessian matrix and computing its eigenvectors $L_i$, preferably the ten first eigenvectors, and (6.2) then generating decoys $X^{decoy}$ wherein such decoy is defined according to equation (16).
For the receptor: (6.1 a) constructing the Hessian matrix and computing its eigenvectors $L_i$, preferably the ten first eigenvectors, and (6.2a) then generating decoys $X^{decoy}$ wherein such decoy is defined according to equation (16).

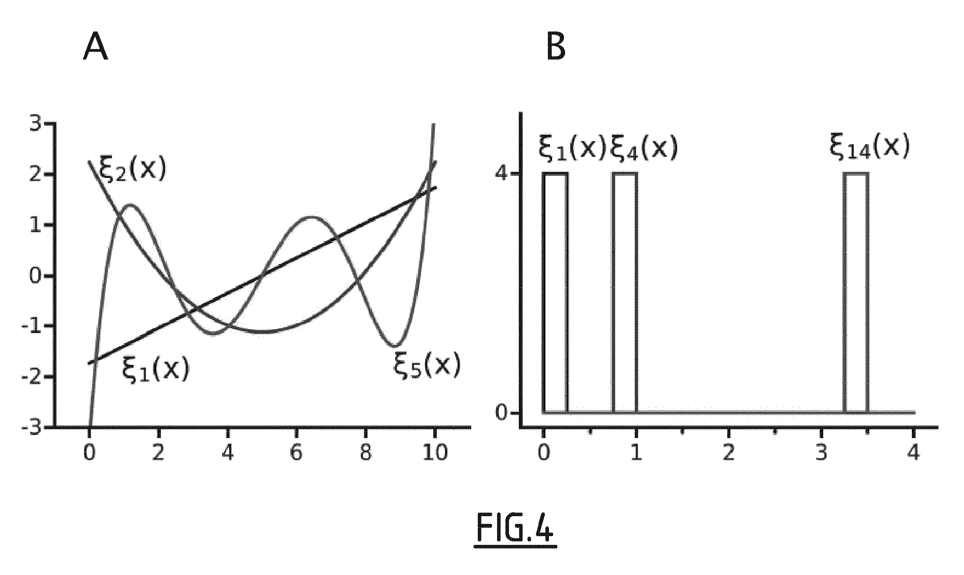

**Figure 4** represents two types of orthogonal functions. Left: shifted Legendre polynomials orthogonal on the interval [0; 10]. Right: shifted rectangular functions.

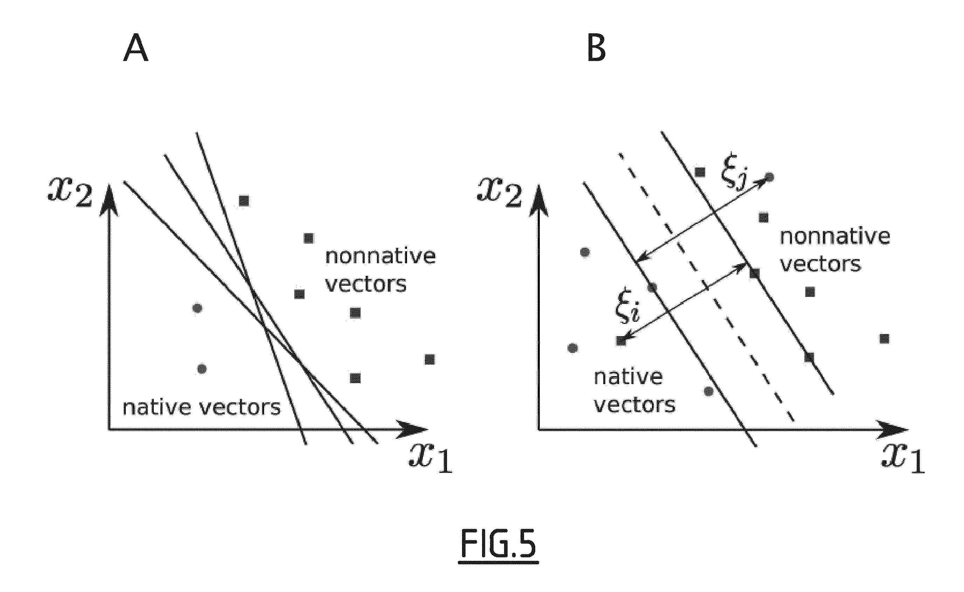

**Figure 5** represents two classes of structure vectors for a single complex. Native structure vectors are plotted as circles. Nonnative structure vectors are plotted as squares. A) The case where infinitely many hyperplanes can separate the two classes. B) The case where no optimal separating hyperplane exists. Slack variables $\xi_i$ and $\xi_j$ for misclassified structure vectors are added, which are the distances to the corresponding margin hyperplanes. The optimal hyperplane, which maximizes the separation between the two classes, is plotted as a dashed line. Two margin hyperplanes are plotted as solid lines.

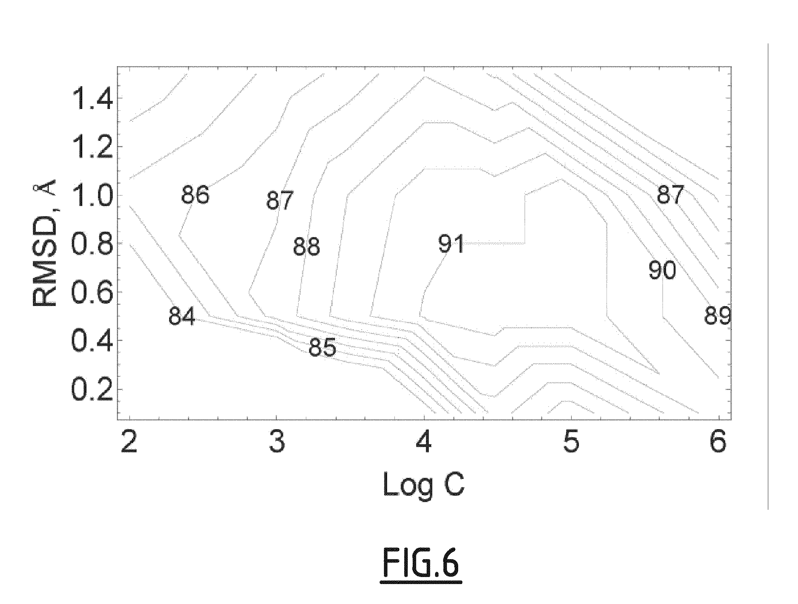

**Figure 6** represents a cross-validation procedure to reveal the optimal RMSD and regularisation parameters for the optimization problem for protein-drug interactions.

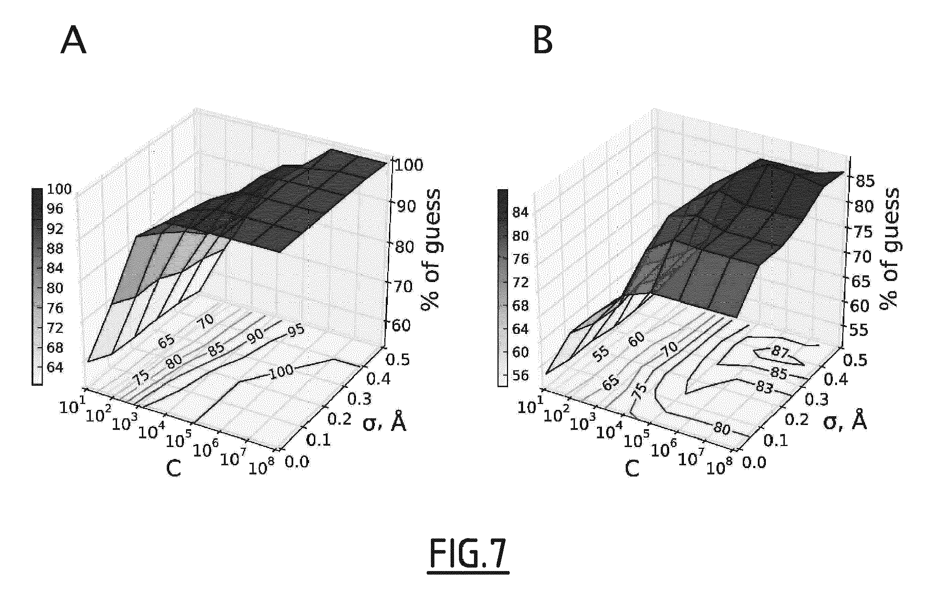

**Figure 7** represents predictive performance of the protein-protein scoring potential as a function of the smoothing parameter $\sigma$ and the regularization parameter C. A) Performance obtained if the scoring functions are trained on the whole database and verified on the same database. B) Performance obtained if the scoring functions are trained on 200 protein complexes and verified on the other 650 complexes from the training database. Here, the best performance is obtained with $\sigma$ = 0...4 A and C = $10^6$ ...$10^7$.

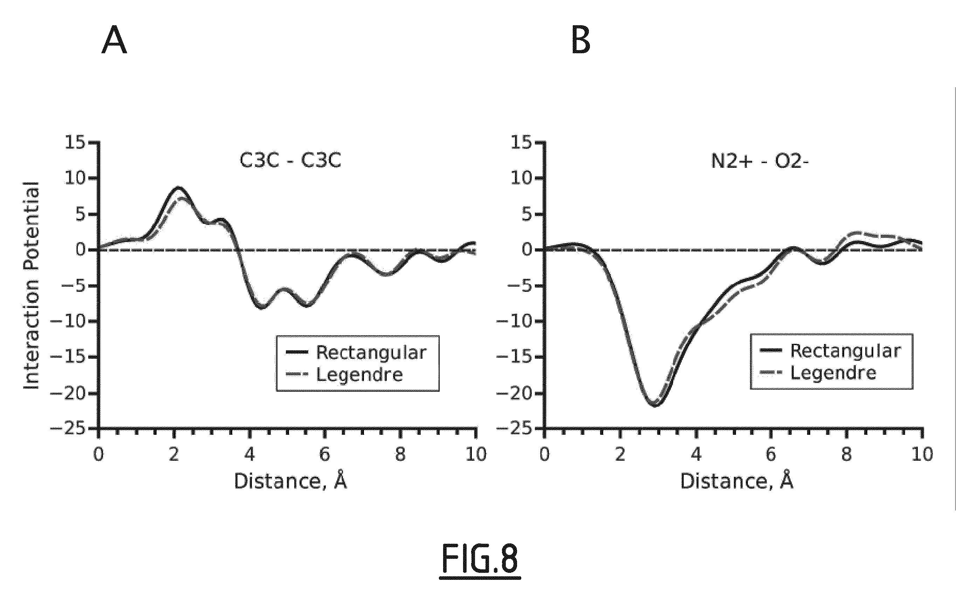

**Figure 8** represents scoring functions trained in two different polynomial bases. Solid lines correspond to the potentials obtained using the rectangular basis functions. Dashed lines correspond to the potentials obtained using the Legendre basis functions. Left: Potential between aliphatic carbons bonded to carbons or hydrogens only. Right: Potential between a guanidine nitrogen with two hydrogens and an oxygen in carboxyl groups.

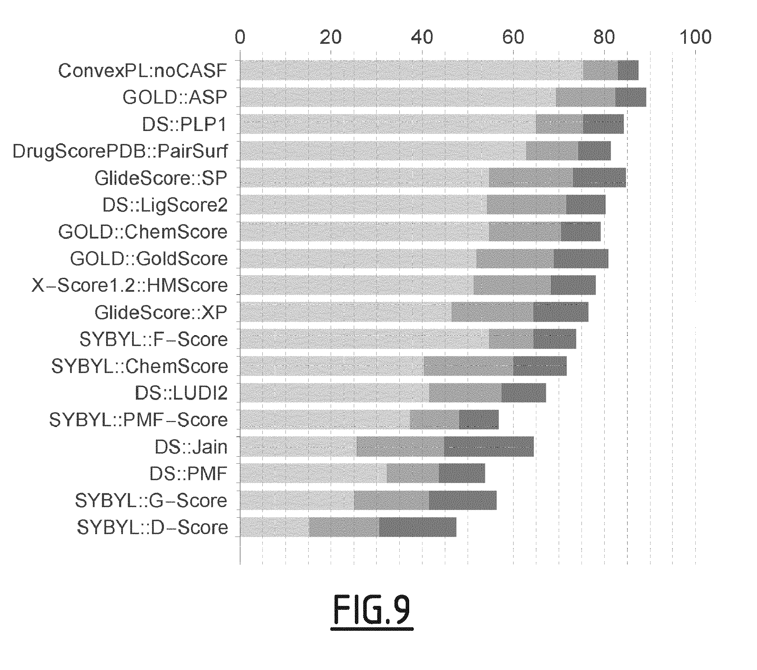

**Figure 9** represents a comparison of the success rates of scoring functions when the best-scored binding pose differs from the true one by RMSD < 1.0 A (light bars) < 2.0 A (darker bars) or < 3.0 A (the darkest bars), respectively. Scoring functions are ranked by success rates when the ligand binding pose is found within RMSD < 2.0 A.

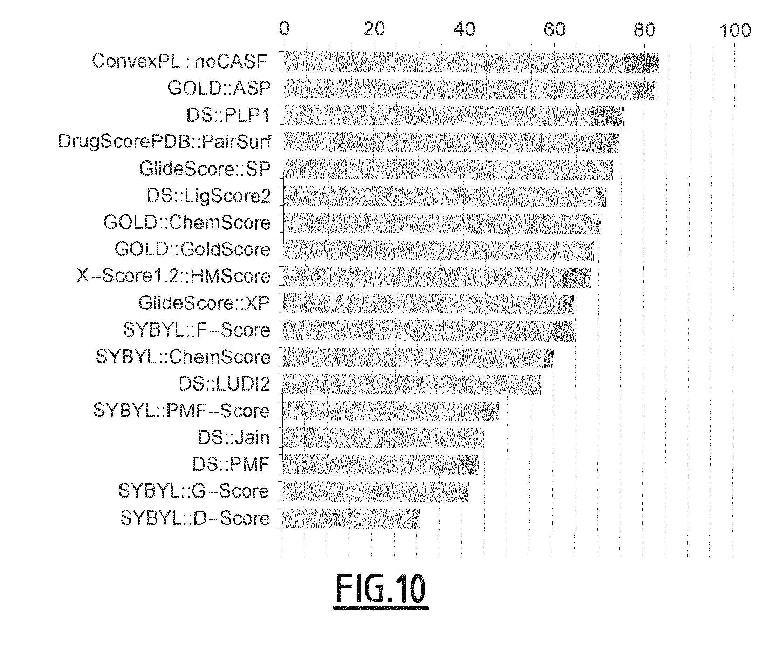

**Figure 10** represents a comparison of the success rates of scoring functions for the cases when the native binding pose is included (dark bars) or not-included (light bars) into the assessment. The acceptance cutoff RMSD is 2.0 A. Scoring functions are ranked by the darker bars.

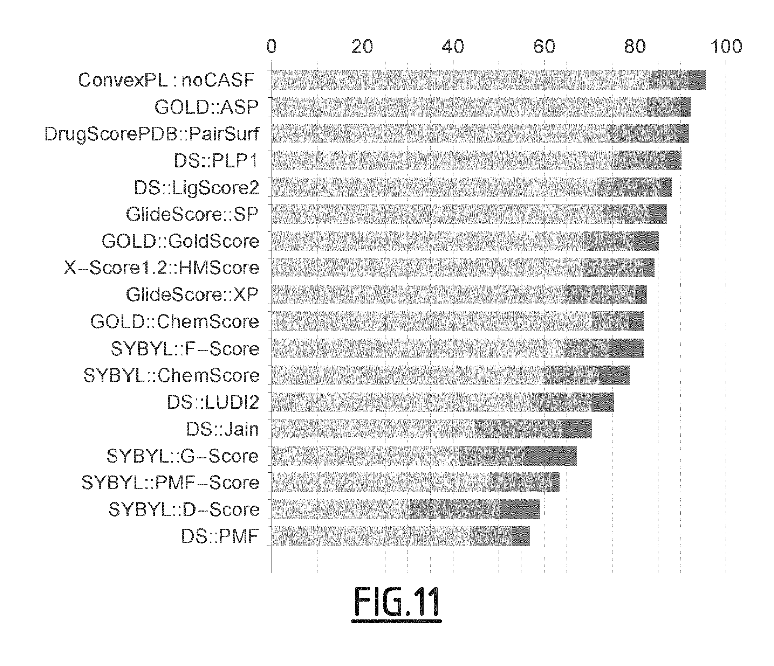

**Figure 11** represents a comparison of the success rates of scoring functions when a ligand binding pose is found within RMSD < 2.0 A from the true one if the top one (light bars), the top two (darker bars), or the top three (the darkest bars) best-scored binding poses are considered. Scoring functions are ranked by success rates when the top three binding poses are considered.

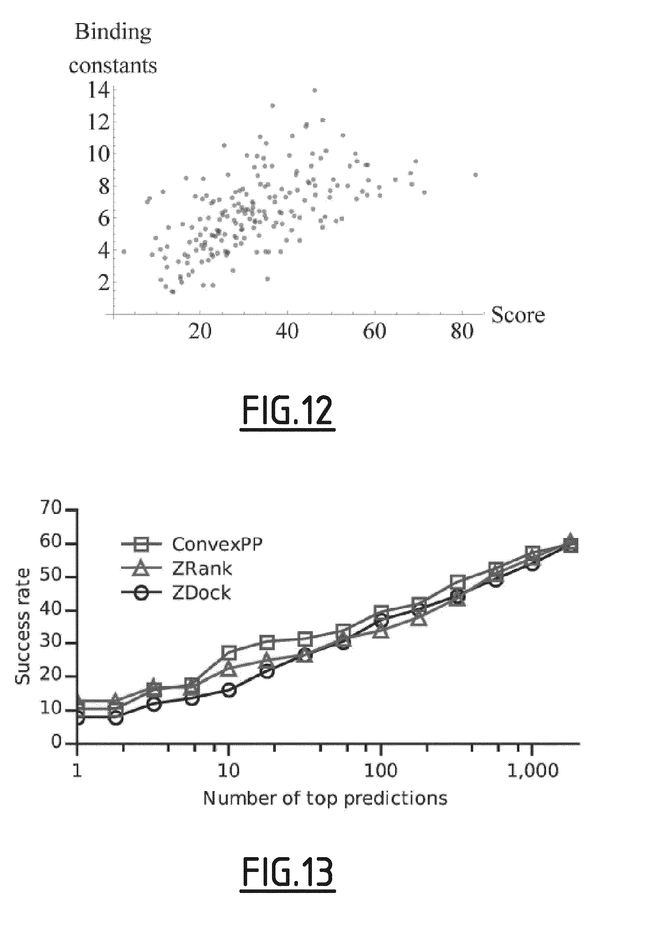

**Figure 12** represents a correlation between experimentally measured binding constants (in -log$K_d$ units) of 195 benchmark complexes and the predicted ones. Pearson correlation coefficient is $R_p$ = 0:59.

**Figure 13** represents a dependence of the success rate on the ZDOCK benchmark on the number of top predictions in consideration for the three methods.

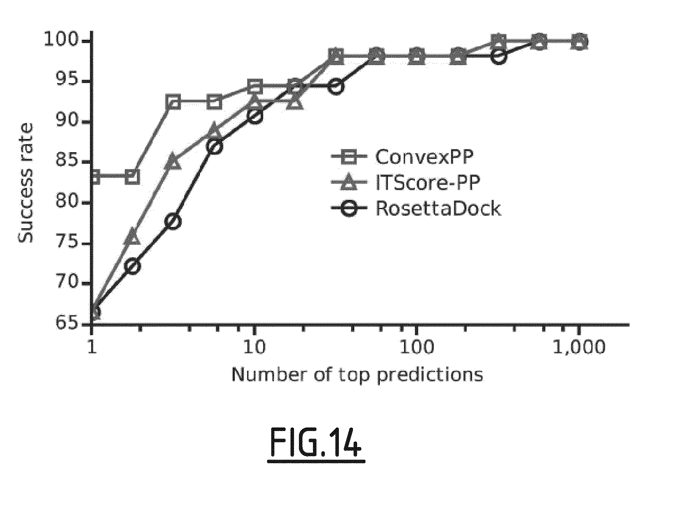

**Figure 14** represents a dependence of the success rate on the RosettaDock benchmark on the number of top predictions in consideration for the three methods.

The invention is described below according to specific examples.

The methods were implemented using the C++ programming language and compiled using g++ compiler version 4.6 with optimization levels -03 and the clang compiler. The programs was ran on a 64-bit Linux Fedora operating system with Intel(R) Xeon(R) CPU X5650 @ 2.67GHz and on a 64-bit Mac OS system version 10.9 with Intel(R) Core i7 CPU @ 2.7GHz.

Example of such method is described below relative to the generation of protein-protein interaction or protein-drug interaction (the drug being a small molecule).

**Example** 1 - Generating Non-Native Receptor-Ligand complexes $P_j^{nonnat}$

Consider $N$ native Receptor-Ligand complex (for example a proteins-protein or protein-drug complex) configurations $P_i^{nat}$, i = 1... N. For each Receptor complex number $i$, D decoys $P_{ij}^{nonnat}$ are generated; j = 1...D, where the first index runs over different protein complexes and the second index runs over generated decoys.

Example 1.1 Protein-protein interactions:

Given each protein near the equilibrium state, the Hessian matrix of the potential energy was constructed and the Fourier subspace for the reduced-basis normal modes computations was computed. The first low-frequency modes from the Fourier basis were picked up to generate different local flexible deformations of the protein complexes. It was decided to exclude the first six modes corresponding to the rigid body motion. More precisely, fifteen decoys for each protein were formed using the linear combinations of modes $\{v_i\}$ as follows:

$$\mathbf{X}^{decoy} = \mathbf{X}^{native} + \sqrt{k_B \mathbf{T}}\, \sum_{i=6}^{15} r_i \frac{v_i}{\omega_i} \qquad (17)$$

Although, generally, the temperature factor is individual for each monomer, we kept it constant for all the monomers and chose its best value. To do so, we scanned through several values of the temperature factor, namely, 5; 10; 20; 40; 60 $(kcal/mol)^{1/2}$, using the cross-validation procedure as detailed below in the text. The temperature factor $\sqrt{k_B T}$ affects the amplitude of the deformation, hence, too large temperatures cause a monomer to deform significantly breaking the covalent bonds. To ensure the absence of non-relevant decoy conformations, we measured the RMSD between the native and the decoy structures for each value of $\sqrt{k_B T}$. Table 1 lists corresponding values of RMSD.

As one can see, indeed, the vast majority of decoys are within 6 Angstrom (A). RMSD with respect to the corresponding native molecule, which means that the normal mode perturbation of the native state (with a given temperature factor) keeps all decoy conformations near-native. At the last step, decoys were combined from two molecules representing one protein complex, resulted in 15 x 15 = 225 decoys. To summarize, five training sets corresponding to different values of the temperature factor $\sqrt{k_B T}$ were composed. Each training set contained 844 blocks representing different non-homologous protein complex, and each block consists of one native structure and 225 decoys generated with normal modes (Note: if protein complexes occurred too large for the normal mode analysis they were removed from the training set). Thus, each training set comprised of 844 x (225 + 1) = 190744 molecule entries, which we used further to derive the statistical scoring function. Normal modes can be also computed in a simpler way using, e.g. the elastic-network, the Gaussian network model, the rotation-translation of blocks method, etc. These methods describe a protein as a set of particles that are interconnected by a network of elastic springs.

Table 1. RMSD corresponded to several temperature factors.

| $\sqrt{k_B T}$, $(kcal/mol)^{1/2}$ | RMSD, Å |
|---|---|
| 5 | 0.507 |
| 10 | 1.015 |
| 20 | 2.034 |
| 40 | 4.058 |
| 60 | 6.083 |

Depending on the level of detail of the model, the particles can correspond to the atoms of the protein, a subset of the atoms, or to representative points such as the center of mass of a residue or a sidechain. The normal modes are then found by diagonalization of the Hessian matrix H, which is the matrix of second derivatives of the potential function with respect to atomic positions, as $H = V \Lambda V^T$.

All generated decoys represent near-native protein structures. Indeed, normal mode oscillations were used to locally deform molecules, however, the orientation of molecules with respect to each other is fixed. Since all decoy molecules slightly differ from the native monomers, as verified by their RMSD values (see Table 1), the interaction interfaces of all decoy complexes undergo moderate changes and keep at least some part of the native contacts. Putting all together, the training set was based only on local information about the native interfaces and no other information was used.

Example 1.2 Protein-ligand interactions:

Generation of the decoy conformations was carried out in the following way. Ligand molecules were considered as rigid bodies and rotated about some axes such that the RMSD distance is kept fixed. To do so, six axes were chosen inside a unit sphere corresponding to its icosahedral tessellation. The weighted RMSD for a pure rotation about axis $n$ by an angle $\alpha$ of a molecule of total mass M with inertia tensor $I$ is:

$$RMSD^2 = \frac{4}{M} \sin^2 \frac{\alpha}{2} I(n), \qquad (18)$$

where the ligand molecule is considered as a rigid body, whose inertia tensor relative to the axis $n$ passing through its center of mass is given as:

$$I(n) = n^T I n \qquad (19)$$

To obtain a decoy with a certain RMSD from the native structure, the latter was first rotated about each rotational axis by $\pm \alpha$ and then translated along coordinate axes by the lengths $\pm RMSD$. Thus, for each native structure, 18 decoy conformations were generated, which means that the total amount of the training structural vectors was $(18 + 1) \times 6004$ = 114,076. In order to determine the optimal value of decoy *RMSD*, the cross-validation process using the training data was carried out. More precisely, the training data was split into two sets, and Eq. (19) was solved while scanning over different parameters of *RMSD* and the regularization constant C. Figure 6 shows the success rates obtained on the second part of the training set. One can see well-distinguished peak of success rate on the 2-fold cross validation test. One can see that in principle, any value of RMSD for decoy generation in [0.2 Å, 1.0 Å] interval can be used, provided the value of the regularization parameter C is chosen correspondingly.

To define types of atoms, one can use Sybyl atom types that are given along with mol2 molecular format and also determined by the OpenBabel molecular library[9], or a more extended set of types, for example the one provided by the fconv library[7].

**Example 2 - Formulating the Optimization Problem**

The aim is to find such a *scoring functional F,* defined for all possible complex structures (the set P), such that for each native complex *i* and its nonnative decoy *j* the following inequality holds:

$$F(P_i^{nat}) < F(P_{ij}^{nonnat}) \qquad (20)$$

Generally, this is a very difficult problem. However, it can be simplified greatly under certain mild assumptions. In order to simplify it, it is assumed the following:

*1.* F depends only on the interface between the receptor and the ligand.

2. F depends only on the distribution of the distances between the interaction sites (the number of site pairs at a certain distance),

$$F(P) \equiv F(n^{11}(r), .., n^{kl}(r), .., n^{mm}(r)) \equiv F(n(r)), \qquad (21)$$

where $n^{kl}(r)$ is, as previously defined, number density functions. For homogeneous systems, such as liquids, functions $n^{kl}(r)$ can be expressed via site-site radial distribution functions $g^{kl}(r)$, which can be obtained experimentally, as $n^{kl}(r)$ = $4\pi r^2 \rho g^{kl}(r)N$, where $p$ is the number density and *N* is the total number of atoms in the system. However, for proteins and small molecules this is not the case.

3. F is a linear functional,

$$F(\alpha n_1(r) + \beta n_2(r)) = \alpha F(n_1(r)) + \beta F(n_2(r)) \qquad (22)$$

One of the simplest functionals F(n(r)) fulfilling these assumptions can be written as:

$$F(n(r)) \equiv F(n^{11}(r), .., n^{kl}(r), .., n^{MM}(r)) = \sum_{k=1}^{M} \sum_{l=k}^{M} \int_0^{r_{max}} n^{kl}(r) U^{kl}(r) dr \qquad (23)$$

It contains unknown functions $U^{kl}(r)$ that can be determined from the training set of native complexes. These functions are defined as *scoring potentials*. Once the scoring functions are known, to compute the value of F, it is needed to specify site-site number densities $n^{kl}(r)$. In practice, they can be calculated as the sum of all *k - l* distances in a given protein complex taking into account *possible inaccuracies in structure determination* via:

$$n^{kl}(r) = \sum_{ij} \frac{1}{\sqrt{2\pi\sigma^2}} e^{-\frac{(r-r_{ij})^2}{2\sigma^2}}, \qquad (24)$$

where each distance distribution is represented by a Gaussian centered at $r_{ij}$ with the standard deviation of $\sigma$. It was assumed that the standard deviation is independent of atom types and thus constant. The sum is taken over all $k$ - $l$ site pairs $i$ and $j$ separated by the distance $r_{ij}$ smaller than a certain threshold $r_{max}$, with site $k$ on the receptor, and site $l$ on the ligand. In the limiting case of standard deviation tending to zero, Eq. (23) turns into a sum over Dirac delta functions. In the present invention the value of $\sigma$ is assumed to be equal for all types of site-site distributions. However, if one has an additional information about individual distance distributions, e.g. Debye-Waller factors, molecular dynamics trajectories, etc., it can be used for a more precise parametrization of standard deviation or even instead of the Gaussian approximation in Eq. (23). Finally, the score of each conformation was computed using the following equation:

$$\text{Score} = \sum_{ij} \Upsilon^{kl}(r_{ij}) \qquad (25)$$

where the sum is taken over all pairs of atoms $i$ and $j$ separated by the distance $r_{ij}$ smaller than the threshold $r_{max}$, with atom $i$ of type $k$ on the receptor, and atom $j$ of type $l$ on the ligand. The function $\gamma^{kl}(r)$ is the Gauss transform of the scoring potential $U^{kl}(x)$:

$$\Upsilon^{kl}(r) = \frac{1}{\sqrt{2\pi\sigma^2}} \int_0^{r_{max}} e^{-\frac{(x-r)^2}{2\sigma^2}} U^{kl}(x)dx \qquad (26)$$

More generally, if the distance distributions have a non-Gaussian shape, $n^{kl}(r) = \sum_{ij} f(r - r_{ij})$, functions $\gamma^{kl}(r)$ will be computed as a convolution $\gamma^{kl} = f * U^{kl}$.

**2.1 Polynomial expansions**

Given a set of functions $\xi_p(r)$ orthogonal on the interval $[r_1;r_2]$ with a nonnegative weight function $\Omega(r)$ such that

$$\int_{r_1}^{r_2} \xi_{p_1}(r)\xi_{p_2}(r)\Omega(r)dr = \delta_{p_1 p_2}, \qquad (27)$$

where $\delta_{p1p2}$ is the Kronecker delta function, the scoring potentials $U_{kl}(r)$ and the number densities $n_{kl}(r)$ can be expanded on the interval $[r_1;r_2]$ as

$$U_{kl}(r) = \sum_p w_p^{kl}\xi_p(r)\sqrt{\Omega(r)}, r \in [r_1;r_2] \qquad (28)$$

$$n_{kl}(r) = \sum_p x_p^{kl}\xi_p(r)\sqrt{\Omega(r)}, r \in [r_1;r_2] \qquad (29)$$

Expansion coefficients $w_p^{kl}$ and $x_p^{kl}$ can be determined from the orthogonality condition (7) as

$$w_p^{kl} = \int_{r_1}^{r_2} U_{kl}(r)\xi_p(r)\sqrt{\Omega(r)}dr \qquad (30)$$

$$x_p^{kl} = \int_{r_1}^{r_2} n_{kl}(r)\xi_p(r)\sqrt{\Omega(r)}dr \qquad (31)$$

Using expansions (8) and (9), the functional F(n(r)) can be rewritten as

$$F(n(r)) = \sum_{k,l}^{M} \int_{0}^{r_{max}} \sum_{p_1} \sum_{p_2} w_{p_1}^{kl} x_{p_2}^{kl} \xi_{p_1}(r)\xi_{p_2}(r)\Omega(r)dr \qquad (32)$$

The present invention was demonstrated using two types of functions $\xi_p(r)$ orthogonal on the interval [0; 10] with a unit weight, (i) shifted Legendre polynomials and (ii) traditionally used shifted rectangular functions. These two types of functions are plotted in Fig. 4.

Other types of orthogonal functions (such as Fourier, Legendre, Hermite or Laguerre) can also be used. If the functions $\xi_p(r)$ are chosen to be negligibly small outside the interval [0; $r_{max}$] or if their interval of orthogonality [$r_1$; $r_2$] coincides with the interval [0; $r_{max}$], as is the case for two sets of the functions according to the invention, then the scoring functional F(n(r)) can be expanded up to some order $P$ as

$$F(n(r)) \approx \sum_{k=1}^{M} \sum_{l=k}^{M} \sum_{p}^{P} w_p^{kl} x_p^{kl} = (\mathbf{w} \cdot \mathbf{x}), \mathbf{w}, \mathbf{x} \in \mathbb{R}^{P \times M \times (M+1)/2} \qquad (33)$$

It is referred the vector w as the *scoring vector* and the vector x as the *structure vector*. Formulas (23) and (30) provide a projection from a protein complex structure into the *scoring space* $\mathbb{R}^{P \times M \times (M+1)/2}$. Using these formulas one can project structural information of each protein complex into a certain structure vector x on $\mathbb{R}^{P \times M \times (M+1)/2}$.

**2.2 Problem Formulation**

Using the expansion of the scoring functional F provided by Eq. (32), one can reformulate the initial scoring problem (19) as follows: given $N$ native structure vectors $\mathbf{x}_i^{nat}$ and $N \times D$ nonnative structure vectors $\mathbf{x}_{ij}^{nonnat}$, find a scoring vector $\mathbf{w} \in \mathbb{R}^{P \times M \times (M+1)/2}$ such that:

$$\forall i = 1...N, \forall j = 1...D(\mathbf{x}_i^{nat} \cdot \mathbf{w}) < (\mathbf{x}_{ij}^{nonnat} \cdot \mathbf{w}), \qquad (34)$$

or equivalently,

$$\forall i = 1...N, \forall j = 1...D([\mathbf{x}_{ij}^{nonnat} - \mathbf{x}_i^{nat}] \cdot \mathbf{w}) > 0), \qquad (35)$$

which is a set of $N \times D$ *half-space equations* in $\mathbb{R}^{P \times M \times (M+1)/2}$ *with N* parallel separation hyperplanes defined by the normal w. Thus, finding the scoring vector is equivalent to finding the common normal to the $N$ planes defined by Eq. (34).

In the training set, some decoy structures can be very close to the native structures. In practice, the native structure is defined as a structure with ligand root-mean-square deviation ($L_{RMSD}$) smaller than a certain threshold distance. Therefore, for each complex, one may have several native structure vectors along with several nonnative structure vectors. Now the question is: how the set of separating hyperplanes shown with common normal w in Fig. 4 is determined? To answer this question it was first considered two special cases presented below.

**2.2.1 Case I. Existence of many solutions**

Fig. 5A is an example of a single complex where infinitely many hyperplanes can separate two classes of structure vectors. A similar example can be easily constructed for the case with multiple complexes. In case of two classes of vectors, Vapnik proposed to use a special kind of separator, the so-called *optimal separating hyperplane*[10], which is unique and maximizes the distance to the closest point from either class. The following *quadratic programming*

*optimization* problem was formulated according to the present invention:

$$\text{Minimize(in } \mathbf{w}, b_j) \quad \frac{1}{2}\mathbf{w} \cdot \mathbf{w}$$
$$\text{Subject to} \qquad y_{ij}\left[\mathbf{w} \cdot \mathbf{x}_{ij} - b_j\right] - 1 \geq 0, i = 1 \ldots N, j = 1 \ldots D \qquad (36)$$

where $y_{ij}$ = -1, when the structure vector $\mathbf{x}_{ij}$ is native and $y_{ij}$ = 1 in the other case. Then Lemma 1 is formulated:

**Lemma 1** *If exists such a linear scoring functional of form (32) that correctly discriminates the native structure vectors for all complexes (equation 34), then the optimal scoring vector is unique and given by the solution of problem (35).*

The scoring vector is optimal in the sense that it maximizes the separation between native and nonnative structure vectors.

Generally, such a linear scoring functional (with a fixed value of the expansion order *P)* may not exist, as demonstrated below. Therefore, the optimization problem (35) has to be modified.

### 2.2.2 Case II. No solution exists

Fig. 5B represents an example where no hyperplane can separate the two classes of the structure vectors of a single complex. For this case, Cortes and Vapnik proposed to relax the condition for the optimal separating hyperplane[11], including an additional term, which is often referred as *loss function,*

$$\text{Minimize(in } \mathbf{w}, b_j) \frac{1}{2}\mathbf{w} \cdot \mathbf{w} + \text{Loss}(\mathbf{w}, \mathbf{x}, \mathbf{b}) \qquad (37)$$

This term can have a general form, however, for practical applications, it is always chosen as a convex function. In the original formulation, this term minimizes the sum of penalties for misclassified vectors. For each decoy set *j = 1... D slack variables* $\xi_{ij}$ have been introduced and are positive for misclassified structure vectors and zero otherwise. A non-zero value of $\xi_{ij}$ allows the structure vector $x_{ij}$ to overcome the inequality condition in Eq. (35) at the cost proportional to the value of $\xi_{ij}$ (see Fig. 5B). The new *soft-margin* quadratic optimization problem then reads:

$$\text{Minimize}\left(in \, \mathbf{w}, b_j, \xi_{ij}\right): \qquad \frac{1}{2}\mathbf{w} \cdot \mathbf{w} + \sum_{ij} C_{ij}\xi_{ij}$$
$$\text{Subject to:} \qquad y_{ij}\left[\mathbf{w} \cdot \mathbf{x}_{ij} - b_j\right] - 1 + \xi_{ij} \geq 0, \quad i = 1 \ldots N, \quad j = 1 \ldots D \qquad (38)$$
$$\xi_{ij} \geq 0$$

The solution of this problem provides a trade-off between how large will be the separation between two classes of the structure vectors of each complex and how many misclassified vectors will be in the solution. Parameters $C_{ij}$ can be regarded as *regularization parameters.* The solution of Eq. (37) tends to maximize the structure vector separation for small values of $C_{ij}$ and minimize the number of misclassified structure vectors for large values of $C_{ij}$. Parameters $C_{ij}$ have been chosen to be different for native and nonnative structure vectors of each complex because fewer native structure vectors should have a larger weight. The following lemma provides the foundation for the numerical scheme used in this work:

**Lemma 2** *The optimal scoring vector is unique and given by the solution of problem (37).*

Here, the scoring vector is optimal in the sense that it maximizes the separation between native and nonnative structure vectors and minimizes the number of misclassified vectors. Regularization parameters $C_{ij}$ in (37) tune the importance of either factors.

The proof of lemmas (1,2) can be found, e.g., in[12]. Overall, the formulation of the optimization problem (37) is very similar to the formulation of the soft-margin *support vector machine* (SVM) problem[11]. Therefore, to solve problem (37), techniques developed for SVM have been used.

### Example 3 - Solving the optimization problem

Properties and solutions of quadratic optimization problems similar to the one stated above (37) have been extensively studied in the theory of convex optimization. They can be solved in dual and primal forms. For instance, using the *Lagrangian* formalism, the optimization problem (37) can be converted into its dual form, and the resulting dual optimization problem is *convex:*

$$\text{Maximize } \mathcal{L}(\lambda_{ij}): \mathcal{L}(\lambda_{ij}) = \sum_{ij} \lambda_{ij} - \frac{1}{2}\sum_{ij}\sum_{kl} y_{ij}y_{kl}\lambda_{ij}\lambda_{kl}\mathbf{x}_{ij}\cdot\mathbf{x}_{kl}$$

$$\text{Subject to: } 0 \leq \lambda_{ij} \leq C_{ij} \tag{39}$$

$$\sum_{j} y_{ij}\lambda_{ij} = 0, \forall i,$$

where the maximization is performed with respect to the *Lagrange multipliers* $\lambda_{ij}$. This dual problem is similar to the the soft-margin SVM optimization problem[11]. Vectors $\mathbf{x}_{ij}$ for which $\lambda_{ij} > 0$ are called *support vectors.* Once the dual problem (38) is solved and the Lagrange multipliers $\lambda_{ij}$ are found, one can express the solution of the original primal problem (37) (the scoring vector) as a linear combination of the support vectors:

$$\mathbf{w} = \sum_{\text{support vectors}} y_{ij}\lambda_{ij}\mathbf{x}_{ij} \tag{40}$$

The problem formulation according to the invention reduces the amount of RAM required by the solver by $N^2$ times.

Therefore, this represents an important technical advantage.

**Example 4 - Generating protein-protein complexes:**

Hex protein docking software has been used.

Initialized Hex exhaustive search algorithm with the radial search step of 1.5 A and expansion order of the shape function equal to 31 has been used. One may use only the shape complementarity energy function from Hex (i.e. electrostatic contribution was omitted). The top 200 clusters, ranked by Hex surface complementarity function, plus the native protein-protein complex conformation (giving in total 201 structures) were then used to evaluate the distance distribution functions (23). Then, the structure vectors using Eq. (30) were computed and labeled as "native" if the root mean square deviation (RMSD) of the corresponding ligand was < 2 A from its native position. Otherwise, the structure vector was labeled as "nonnative" or "decoy". On average, about 2.5 native structure vectors (and, correspondingly, about 198.5 nonnative structure vectors) per protein-protein complex were obtained. To each structure vector $\mathbf{x}_{ij}$ a regularization parameter $C_{ij}$ was assigned according to

$$C_{ij}^{native} = CD_j^{nonnative}/D_j$$
$$C_{ij}^{nonnative} = CD_j^{native}/D_j' \tag{41}$$

where $D_j$ is the total number of structure vectors for each protein-protein complex (201 in our case), $D_j^{native}$ is the number of native structure vectors for complex $j$ and $D_j^{nonnative}$ is the number of nonnative structure vectors for complex $j$. The same procedure was respected for each protein-protein complex from the training database. In this example, M = 20 atom-centered interaction sites based on the atom types definitions provided by Huang and Zou[13]. These atom types were defined by the classification of all heavy atoms in 20 standard amino acids according to their element symbol, aromaticity, hybridization, and polarity. These 20 atom types result in total of M x (M+1) = 210 pair potentials. The training set has several proteins homologous to the ones from the two widely used docking benchmarks, Rosetta, and Zdock, which were used below to validate the results of the invention. Two protein complexes were defined to be homologous if for each chain in the first complex there is a chain in the second complex with sequence identity more than 60%. We determined the sequence identity using FASTA36 program.

**Example 5 - Generating protein-drug complexes:**

PDBBind database[14] provides experimentally measured binding affinity data for the complexes deposited in the Protein Data Bank. The "general set" of release PDBBind 2011 contains binding data ($K_d$, $K_i$ & $IC$50 values) and three-dimensional structures of resolution equal to or better than 2.5 Å for 6051 protein-ligand complexes. This information was used in order to derive the scoring function for protein-drug interactions.

### Example 6 - Training set for protein-protein interactions

To predict protein-protein interactions we used the training database of 851 non-redundant protein-protein complex structures collected by Huang and Zou[13]. This database contains protein-protein complexes extracted from the PDB[15] and includes 655 homodimers and 196 heterodimers. Three PDB structures from the original training database were updated: 2Q33 supersedes 1N98, 2ZOY supersedes 1V7B, and 3KKJ supersedes 1YVV. The training database contains only crystal dimeric structures determined by X-ray crystallography at resolution better than 2.5 Å. Each chain of the dimeric structure has at least 10 amino acids, and the number of interacting residue pairs (as defined as having at least 1 heavy atom within 4.5 A) is at least 30. Each protein-protein interface consists only of 20 standard amino acids. No homologous complexes were included in the training database. Two protein complexes were regarded as homologues if the sequence identity between receptor-receptor pairs and between ligand-ligand pairs was > 70%. Finally, Huang and Zou manually inspected the training database and left only those structures that had no artifacts of crystallization.

The algorithm of the invention requires as input native and nonnative structure vectors (see, e.g., equation (14)). Native structure vectors can be computed from the native protein-protein contacts in the training database using equation (11). However, for the computation of the nonnative structure vectors for each protein-protein complex from the training database, decoys were generated for each complex. Since the optimization algorithm of the invention is very general and has no special requirements for nonnative protein-protein contacts, nonnative protein-protein were generated by "rolling" a smaller protein (ligand) over the surface of a bigger protein (receptor) using the Hex protein docking software[8]. To do so, Hex exhaustive search algorithm initialized with the radial search step of 1.5 Å and expansion order of the shape function equal to 31. Only the shape complementarity energy function from Hex (i.e., electrostatic contribution was omitted) was used. The top 200 clusters, ranked by Hex surface complementarity function, plus the native protein-protein complex conformation (giving a total of 201 structures) were then used to evaluate the distance distribution functions (23). Then, the structure vectors using Eq. (11) were computed and labeled according to example 4.

For the optimization of protein-protein interactions, we used the following parameters, $\sigma = 0{:}4$ Angstrom (A), $C = 10^5$. The maximum expansion order P was set to P = 40. Figure 8 shows two examples of the scoring function $\gamma^{kl}(r)$ (see Eq. (5)).

### Example 7 - Training set for protein-drug interactions

PDBBind database provides experimentally measured binding affinity data for the complexes deposited in the Protein Data Bank. The "general set" of release PDBBind 2011 contains binding data ($K_d, K_i$ & $IC50$ values) and three-dimensional structures of resolution equal to or better than 2.5 Å for 6051 protein-ligand complexes. This information was used in order to derive the scoring function for protein-drug interactions.

Generation of the decoy conformations was carried out in the following way. Ligand molecules were considered as rigid bodies and rotated about some axes such that the RMSD distance is kept fixed. This generation of decoys was performed according to example 5.

For the optimization of protein-drug interactions, the following parameters, were used: $\sigma = 0.4$ Angstrom (A), $C = 10^5$ and RMSD = 0.6 Angstrom (A). The maximum expansion order P was set to P = 25.

### Example 8 - Gradient-based structure optimization

Note that orthogonal polynomials used for the expansion of the scoring potentials (Eq. 8) might be non-smooth functions, e.g. rectangular polynomials. Thus, generally, the scoring potentials $U_{kl}(r)$ could be not differentiable. However, thanks to the applied Gauss transform, functions $Y^{kl}(r)$ (Eq. (4)) are smooth as a convolution of analytic locally integrable functions. This fact allows extending the functionality of functions $Y^{kl}(r)$ from the scoring to the structure optimization using their first or higher-order derivatives. More accurately, for a given k-l pair of atoms at a distance $r_{ij}$, the negative gradient $-\nabla Y^{kl}(r_{ij})$ equals to the force acting on the atoms in this pair. Thus, the set of derived functions $Y^{kl}(r)$ could be used in a force-field manner, optimizing the structure of a particular complex until a local minimum is reached, provided $\nabla Y^{kl}(r_{ij}) = 0$ for each pair of atoms.

Since special calibration of the potential functions is required to retain the structure integrity of a complex, more relevant application would be a *rigid-body* optimization, where instead of force minimization over each pair of atoms, one minimizes the net force acting on the complex. Thus, at a local minimum, $\sum_{k,l}\sum_{i,j}\nabla Y^{kl} = 0$ holds. Rigid-body optimization with functions $Y^{kl}(r)$ could be useful in a local rigid-body minimization as a refinement step to process docking predictions. It was shown that such refinement could improve docking predictions dramatically. By contrast to our functions $Y^{kl}(r)$, most of modern statistical pair-wise potentials are not differentiable (ITScore, DOPE, DFIRE, RAPDF, etc.). Thereby, to perform optimization with such potentials one either smooth them a-posterior, which worsen the potential quality, or uses various derivative-free optimization strategies, e.g. Nelder-Mead or Powell methods and their modifications, where the convergence rate is much slower compared to the first- or higher-order optimization strategies.

**Example 9 - Cross validation studies**

To tune free regularization parameters C and $\sigma$, and also the value of the RMSD parameter used during decoy generation, we carried out a series of cross-validation computational experiments, where we split the training dataset into two parts following the training on the first part and validation on the second part. Results of the cross-validation for protein-protein interactions are shown in Fig. 7. Here, the best performance is achieved with $\sigma = 0.4$ Å, and C = $10^5 ...10^6$. Results of the cross-validation for protein-drug interactions are shown in Fig. 6. We can see that the optimum parameters belong to the range of C = $10^4 ...10^6$ and RMSD = 0.4 ...1 Å. For the production run optimization, we used the following parameters, $\sigma = 0.4$ Å, C = $10^5$ Å and RMSD = 0.6 Å.

The width of the Gaussian parameter $\sigma$ dictates the number of polynomial coefficients sufficient to encode the shape of the potential. More precisely, we let the maximum expansion order P to be $P = r_{max}/\sigma$. Using the values of $r_{max}$ = 10 Å and $\sigma = 0.4$ Å, we concluded that the maximum expansion order is P = 25. Using the Legendre polynomial basis, we have numerically verified that higher expansion orders do not contribute to the quality of the reconstructed potentials, provided that the parameters $r_{max}$ and $\sigma$ are kept constant.

**Example 10 - Ranking of protein-protein and protein-drug structures**

If structure optimization is not required, as it happens in scoring of decoys generated by other docking programs, then *ranking* is performed using Eq. (12). More precisely, for each structure of a protein-protein or a protein-grid complex, one computes the structure vectors $x_p^{kl}$ using Eq. (11). Then, these structure vectors are multiplied with the pre-computed scoring vectors $w_p^{kl}$ according to Eq. (12) and a linear approximation of the binding free energy is obtained.

Now, structures of the complexes can be ranked according to this free energy approximation. If structure optimization is desired, in practice we use Eqs. (4-5) for the gradient-based structure optimization. During the optimization, the gradient of the scoring function (4) is computed with respect to six rigid-body coordinates of the receptor and the ligand. Then, the structure is iteratively optimized until a certain convergence is achieved. Finally, different structures are ranked according to the scores of the optimized binding poses.

Here one should note that the binding free energy F and the binding affinity terms are synonymous. Both are connected to the experimentally measured dissociation constant Kd as F = RT logKd / c, where R is the ideal gas constant, T is temperature and the standard reference concentration c = 1 mol / L.

**Example 11- Results for protein-drug interactions**

**11.1 Docking power**

The first general method of assessment of a scoring function is to see how well it can predict the true binding pose. More precisely, if the best ranked ligand pose is close enough (within RMSD range of 1.0, 2.0 or 3.0 A) to the known true one, the scoring function is said to guess it correctly within a certain RMSD threshold. Success rate of the scoring function according to the invention in comparison to the others is shown in Figure 9. Figure 10 represents the difference in results, when native conformation is included or excluded from the decoy set. As one can see, the difference is not more than 5% for all the scoring functions, which is not very significant. Therefore, the native pose was included into the decoy sets from the benchmark in order to be able to compare the performance to the results published previously. In the comparisons above, only the best ranked ligand poses were considered. Practically, during prediction of the binding pose, it is appropriate to submit few poses. Figure 11 shows success rates in cases when one, two or three best ranked poses are considered. For many scoring functions one can notice a significant increase in the prediction power when several poses are considered in comparison to Figure 9. Another representation of docking power evaluation results that includes success rates of the DSX[16] scoring functions is given in Table 2. Results of DSX cited from[16] and the rest (excluding ConvexPL) - from[1]. The last column corresponds to the success rate of finding the top ranked ligand pose within RMSD $\leq 2.0$ Å from the crystallographically determined one, when this true one is excluded from the decoy set.

ConvexPL is the scoring function according to the invention.

Table 2: Success rates in docking power assessment.

| | Crystal structure on | | ≤ 2.0 Å pose on | | |
|---|---|---|---|---|---|
| Scoring function | Top 1 pose | Top 5 poses | Top 1 pose | Top 5 poses | Top 1 pose no cryst. |
| **ConvexPL** | 55.9 | 80.5 | 83.1 | 97.8 | 75.4 |
| DS::Jain | 1.5 | 15.4 | 44.8 | 79.2 | 44.8 |
| DS::LigScore2 | 17.9 | 49.7 | 71.6 | 92.9 | 69.4 |
| DS::LUDI2 | 9.7 | 29.2 | 57.4 | 83.6 | 56.8 |
| DS::PLP1 | 40.5 | 75.9 | 75.4 | 97.3 | 68.3 |
| DS::PMF | 19.5 | 44.1 | 43.7 | 67.2 | 39.3 |
| DrugScoreCSD::Pair | 50.3 | 79.5 | 58.5 | 94.0 | 25.7 |
| DrugScoreCSD::PairSurf | 44.6 | 80.0 | 54.1 | 95.6 | 25.1 |
| DrugScorePDB::Pair | 40.0 | 73.8 | 74.3 | 93.4 | 68.9 |
| DrugScorePDB::PairSurf | 39.5 | 74.9 | 74.3 | 95.1 | 69.4 |
| DrugScorePDB::Surf | 3.6 | 20.0 | 32.8 | 80.3 | 32.2 |
| DSX$^{PDB}$::PairSR | 51.8 | 77.9 | 84.7 | 95.6 | 78.7 |
| DSX$^{CSD}$::All | 52.8 | 77.9 | 85.2 | 96.2 | 79.2 |
| GOLD::ASP | 36.9 | 71.8 | 82.5 | 95.6 | 77.6 |
| GOLD::ChemScore | 17.9 | 50.8 | 70.5 | 86.9 | 69.4 |
| GOLD::GoldScore | 8.2 | 28.7 | 68.9 | 89.6 | 68.3 |
| GlideScore::SP | 18.5 | 50.3 | 73.2 | 93.4 | 72.7 |
| SYBYL::F-Score | 21.5 | 49.2 | 64.5 | 90.7 | 60.1 |
| X-Score1.2 | 32.3 | 64.6 | 67.2 | 91.3 | 63.4 |
| X-Score1.2::HMScore | 30.3 | 57.9 | 68.3 | 90.7 | 62.3 |

## 11.2. Scoring power

The second evaluation criterion for a scoring function is how well it can predict the binding affinity of a protein-drug complex. Table 3 shows the correlations between true binding constants ($K_d$) and the binding scores obtained with the scoring function, which corresponds to Table 2 from[1].

The problem of predicting correct binding affinity and the next one, ligand ranking, are far more challenging problems, than ligand docking. There is a correlation between the size of a ligand (the number of heavy atoms - NHA in Table 3) and its binding affinity. For the test set, the value of the Pearson correlation for the function according to the invention ("ConvexPL") is 0.431. Such a simple measure as ligand size provides a better correlation coefficient than some of scoring functions. Even scoring functions that show the best results in docking power do not achieve high correlations between binding scores and true binding affinities. And vice versa, if a function shows good correlation, it could still achieve modest results in docking.

As mentioned above, the test set is highly diverse - there is a big difference between the highest and the lowest binding affinity of complexes included in the set, as it is evident from Figure 10. Probably this is one of the reasons of such moderate success rates of all the scoring functions, and if one considers only particular family of protein-ligand complexes, better results can be achieved. See[1] and their additional test sets.

The presence of 195 test protein-ligand complexes in the training sets can be an issue for some functions. To assess it, the success rates were provided when the test set is included to the training set or excluded from it in Table 3 for ConvexPL and X-Score (version with excluded test set is named 1.3) The best three results are shown by empirical-based X-Score, the knowledge-based DSXCSD::All and ConvexPL.

Table 3: Correlations between the experimentally measured binding constants and the binding scores

| Scoring function | $R_p$ | $R_s$ |
|---|---|---|
| X-Score::HMScore | 0.644 | 0.705 |
| DSXCSD::All | 0.609 | - |
| **ConvexPL** | **0.591** | **0.648** |
| **ConvexPL (test set excluded**) | **0.587** | **0.642** |
| DSXPDB::PairSR | 0.571 | - |
| DrugScoreCSD | 0.569 | 0.627 |
| SYBYL::ChemScore | 0.555 | 0.585 |
| DS::PLP1 | 0.545 | 0.588 |
| GOLD::ASP | 0.534 | 0.577 |
| SYBYL::G-Score | 0.492 | 0.536 |
| DS::LUDI3 | 0.487 | 0.478 |
| DS::LigScore2 | 0.464 | 0.507 |
| GlideScore-XP | 0.457 | 0.435 |
| DS::PMF | 0.445 | 0.448 |
| GOLD::ChemScore | 0.441 | 0.452 |
| NHA | 0.431 | 0.517 |
| SYBYL::D-Score | 0.392 | 0.447 |
| DS::Jain | 0.316 | 0.346 |
| GOLD::GoldScore | 0.295 | 0.322 |
| SYBYL::PMF-Score | 0.268 | 0.273 |
| SYBYL::F-Score | 0.216 | 0.243 |

## 11.3 Ranking power

Finally, the last assessment criterion for a scoring function, studied by[1] is the ligand ranking power. Let's consider a given protein target and a list of ligand molecules. Cheng, et al. define the ranking power of a scoring function as the ability to correctly rank the known ligands bound to a common target by their binding affinities when their true binding modes are known.

Table 4 shows the success rates of several scoring functions. The best four functions for ranking are X-Score, DSXCSD::All, DS::PLP2, ConvexPL. Success rates of these top functions are comparable to the success rates in the scoring power assessment. This fact seems interesting, because one could expect that the ligand ranking is an easier problem than scoring. Again, the best results are achieved by the empirical-based function as X-Score and DS::PLP2. Excluding the 195 test complexes from the training set of the function according to the invention leads to the improvement of the success rate by about 1.6% (ConvexPL test set excluded).

Table 4: Success rates in the ligand ranking assessment.

| Scoring function | Success rate % |
|---|---|
| X-Score::HSScore | 58.5 |
| DSXCSD::All | 55.4 |
| DS::PLP2 | 53.8 |
| **ConvexPL (test set excluded**) | **52.3** |
| DSXPDB::PairSR | 52.3 |

(continued)

| Scoring function | Success rate % |
|---|---|
| DrugScoreCSD | 52.3 |
| **ConvexPL** | **50.7** |
| SYBYL::ChemScore | 47.7 |
| SYBYL::D-Score | 46.2 |
| SYBYL::G-Score | 46.2 |
| GOLD::ASP | 43.1 |
| DS::LUDI3 | 43.1 |
| DS::Jain | 41.5 |
| DS::PMF | 41.5 |
| SYBYL::PMF-Score | 38.5 |
| GOLD::ChemScore | 36.9 |
| DS::LigScore2 | 35.4 |
| GlideScore-XP | 33.8 |
| NHA | 32.3 |
| SYBYL::F-Score | 29.2 |
| GOLD::GoldScore | 23.1 |

Parameters obtained with the invention outperform all academic and industrial scoring functions (35 different in total) as presented on Figures 9, 10 and 11.

The present invention also ensures not only a superb predictive power of docking poses, but also a very good correlation between the scores and the binding affinity data

**Example 12 - Results for protein-protein interactions**

**12.1 ZDOCK Benchmark**

We tested the ConvexPP scoring function on the protein-protein docking benchmark version 3.0. It consists of 124 crystallographic structures of protein-protein complexes extracted from the PDB database[17]. These are divided into three groups: rigid, medium and difficult cases. The division criteria is the scale of conformational changes of the proteins upon binding: from minor changes in rigid cases to the major ones in difficult cases. The non-redundancy of the benchmark was set at the level of family-family pairs.

The decoys for the scoring were generated using ZDOCK 3.0 with the sampling step equal to 6 degrees. We call this set of docking position *ZDOCK benchmark.* The docking program ZDOCK 3.0 generates the rigid-body protein-protein docking predictions with the corresponding scores. Scoring function used in this program includes shape complementarity, statistical pair potentials and electrostatics. ZRANK is the program for reranking the ZDOCK3.0 predictions. In addition to the factors used in ZDOCK3.0, it computes detailed electrostatics, estimates desolvation and uses additional Van-der-Waals potential to re-score the decoys. The benchmark 3.0 has several complexes homologous to certain protein complexes in the training set. Therefore, we trained our potential both excluding homologs from the training set and leaving it unchanged. Table 5 shows results of ZDOCK3.0, ZRANK and our scoring functions on the ZDOCK3.0 benchmark.

2000 decoys generated by ZDOCK3.0 were ranked with the original ZDOCK function, ZRANK and the scoring potentials according to the invention. A hit is a predicted near-native decoy with $I_{RMSD}$ less than 2.5 Å. The $I_{RMSD}$ parameter is the RMSD of the interface region between the predicted and native structures after optimal superimposition of the backbone atoms of the interface residues. A residue is considered as the interface residue if any atom of this residue is within 10 Å from the other partner. The number of hits when only the top one prediction considered (Top1) obtained by ZRANK is higher than the one obtained by ConvexPP potentials (15 vs 12 hits). Although if top 10 predictions were considered, the scoring function according to the invention outperforms ZRANK (32 vs 26 hits). Excluding homologs from the training set results in a slight improvement of the results (Table 5).

Table 5: ZDock benchmark 3.0 results. Three scoring functions are compared, ZDock, ZRank, and ConvexPP. Proteins homologous to the ones in the training set are shown in bold. Absence of hits among the first 2000 predictions is shown with hyphens. The $I_{RMSD}$ parameter represents the quality of a pose, which is the RMSD of the backbone atoms of the ligand after the receptors in the native and the decoy conformations have been optimally superimposed. The IRMSD parameter is the RMSD of the interface region between the predicted and native structures after optimal superimposition of the backbone atoms of the interface residues. A residue is considered as the interface residue if any atom of this residue is within 10 Å from the other partner. The fnat parameter is the ratio of the number of native residue-residue contacts in the predicted complex to the number of residue-residue contacts in the crystal structure.

Table 5: ZDock benchmark 3.0 results

| Complex | ZDock | | | | ZRank | | | | ConvexPP | | | |
|---|---|---|---|---|---|---|---|---|---|---|---|---|
| | Rank | $L_{RMSD}$ | $I_{RMSD}$ | $f_{nat}$ | Rank | $L_{RMSD}$ | $I_{RMSD}$ | $f_{nat}$ | Rank | $L_{RMSD}$ | $I_{RMSD}$ | $f_{nat}$ |
| Rigid-Body | | | | | | | | | | | | |
| 1AHW | 54 | 8.30 | 1.58 | 0.60 | 175 | 2.68 | 1.26 | 0.72 | 547 | 2.14 | 0.91 | 0.79 |
| 1BVK | - | - | - | - | - | - | - | - | - | - | - | - |
| 1DQJ | - | - | - | - | - | - | - | - | - | - | - | - |
| 1 E6J | 1 | 3.80 | 1.59 | 0.64 | 12 | 6.06 | 2.35 | 0.40 | 35 | 4.21 | 2.26 | 0.48 |
| 1JPS | 1 | 3.90 | 1.04 | 0.70 | 254 | 4.32 | 1.26 | 0.65 | 62 | 4.14 | 1.11 | 0.78 |
| 1MLC | 5 | 4.61 | 1.14 | 0.36 | 54 | 4.61 | 1.14 | 0.36 | 5 | 4.70 | 1.12 | 0.39 |
| 1VFB | 997 | 10.89 | 2.48 | 0.30 | 798 | 0.89 | 2.48 | 0.30 | 1239 | 10.89 | 2.48 | 0.30 |
| 1WEJ | 2 | 2.44 | 0.79 | 0.91 | 41 | 2.44 | 0.79 | 0.91 | 1 | 4.13 | 1.30 | 0.75 |
| 2FD6 | 15 | 18.67 | 2.42 | 0.73 | 9 | 9.76 | 2.42 | 0.80 | 8 | 15.65 | 2.16 | 0.80 |
| 2I25 | 1534 | 7.92 | 2.21 | 0.36 | 1 | 4.45 | 1.87 | 0.33 | 83 | 4.45 | 1.87 | 0.33 |
| 2VIS | 8 | 27.81 | 2.02 | 0.63 | 150 | 6.31 | 2.18 | 0.57 | 617 | 23.89 | 2.37 | 0.43 |
| 1BJ1 | 19 | 6.29 | 1.19 | 0.62 | 1 | 4.22 | 0.97 | 0.88 | 2 | 2.82 | 0.98 | 0.86 |
| 1FSK | 1 | 2.69 | 1.04 | 0.91 | 3 | 1.39 | 0.65 | 0.93 | 3 | 3.98 | 1.39 | 0.81 |
| 1I9R | 40 | 3.07 | 1.53 | 0.79 | 493 | 3.07 | 1.53 | 0.79 | 462 | 16.85 | 2.30 | 0.48 |
| 1IQD | 169 | 5.25 | 1.01 | 0.60 | 3 | 3.08 | 0.88 | 0.73 | 16 | 4.20 | 0.97 | 0.67 |
| 1K4C | 587 | 7.42 | 1.67 | 0.43 | 1615 | 9.12 | 1.64 | 0.45 | 242 | 5.78 | 1.31 | 0.62 |
| 1KXQ | 14 | 2.04 | 1.28 | 0.70 | 1 | 1.75 | 0.93 | 0.93 | 1 | 3.06 | 1.04 | 0.88 |
| 1NCA | 14 | 2.85 | 0.92 | 0.83 | 150 | 1.75 | 0.97 | 0.76 | 12 | 4.50 | 1.38 | 0.86 |
| 1NSN | 473 | 4.95 | 2.00 | 0.50 | 728 | 2.41 | 1.06 | 0.79 | 636 | 4.95 | 2.00 | 0.50 |
| 1QFW | 192 | 5.05 | 1.24 | 0.71 | 310 | 4.21 | 1.41 | 0.77 | 1315 | 5.12 | 1.35 | 0.73 |
| 1QFW | 192 | 5.05 | 1.24 | 0.71 | 310 | 4.21 | 1.41 | 0.77 | 1315 | 5.12 | 1.35 | 0.73 |
| 2JEL | 1239 | 6.12 | 1.90 | 0.55 | 223 | 6.12 | 1.90 | 0.55 | 957 | 6.83 | 2.30 | 0.31 |
| **1AVX** | **11** | **7.49** | **1.61** | **0.56** | **7** | **6.73** | **1.86** | **0.54** | **3** | **4.85** | **2.23** | **0.39** |
| **1AY7** | **74** | **3.82** | **2.13** | **0.52** | **468** | **3.82** | **2.13** | **0.52** | **185** | **5.73** | **1.82** | **0.45** |
| **1BVN** | **16** | **2.60** | **1.54** | **0.43** | **1** | **3.74** | **1.85** | **0.46** | **3** | **4.09** | **1.74** | **0.50** |
| **1CGI** | **89** | **4.27** | **2.34** | **0.43** | **14** | **4.27** | **2.34** | **0.43** | **61** | **3.20** | **2.30** | **0.49** |
| **1D6R** | - | - | - | - | - | - | - | - | - | - | - | - |
| 1 DFJ | 2 | 5.12 | 2.08 | 0.55 | 1 | 3.82 | 1.87 | 0.52 | 1 | 5.97 | 2.42 | 0.50 |
| 1E6E | 5 | 2.97 | 2.00 | 0.42 | 15 | 2.98 | 1.72 | 0.53 | 9 | 4.01 | 2.41 | 0.42 |

(continued)

| Complex | ZDock | | | | ZRank | | | | ConvexPP | | | |
|---|---|---|---|---|---|---|---|---|---|---|---|---|
| | Rank | $L_{RMSD}$ | $I_{RMSD}$ | $f_{nat}$ | Rank | $L_{RMSD}$ | $I_{RMSD}$ | $f_{nat}$ | Rank | $L_{RMSD}$ | $I_{RMSD}$ | $f_{nat}$ |
| 1 EAW | 1 | 9.32 | 2.48 | 0.46 | 42 | 9.32 | 2.48 | 0.46 | 1 | 2.60 | 1.03 | 0.70 |
| 1 EWY | 21 | 3.16 | 1.74 | 0.56 | 9 | 3.32 | 1.88 | 0.61 | 21 | 3.16 | 1.74 | 0.56 |
| 1EZU | - | - | - | - | - | - | - | - | - | - | - | - |
| 1F34 | 62 | 7.51 | 2.34 | 0.41 | 34 | 5.95 | 2.46 | 0.49 | 38 | 3.41 | 1.45 | 0.54 |
| 1HIA | - | - | - | - | - | - | - | - | - | - | - | - |
| 1MAH | 3 | 2.77 | 1.12 | 0.72 | 1 | 2.77 | 1.12 | 0.72 | 1 | 3.64 | 1.26 | 0.69 |
| 1N8O | 92 | 4.60 | 1.51 | 0.60 | 1 | 5.15 | 1.51 | 0.68 | 1 | 2.94 | 1.24 | 0.74 |
| **1OPH** | - | - | - | - | - | - | - | - | - | - | - | - |
| **1PPE** | **1** | **1.84** | **0.77** | **0.79** | **1** | **2.25** | **0.86** | **0.83** | **1** | **4.62** | **1.52** | **0.71** |
| **1ROR** | **70** | **1.83** | **0.71** | **0.74** | **178** | **1.32** | **0.74** | **0.60** | **2** | **8.36** | **2.46** | **0.40** |
| **1TMQ** | **71** | **4.92** | **2.08** | **0.35** | **61** | **3.61** | **1.49** | **0.60** | **8** | **6.11** | **1.97** | **0.45** |
| 1UDI | - | - | - | - | - | - | - | - | - | - | - | - |
| 1YVB | 38 | 12.34 | 2.32 | 0.54 | 6 | 7.33 | 1.92 | 0.71 | 8 | 7.33 | 1.92 | 0.71 |
| 2B42 | 10 | 6.17 | 1.17 | 0.89 | 1 | 6.17 | 1.17 | 0.89 | 8 | 9.44 | 2.23 | 0.43 |
| 2MTA | 748 | 4.86 | 2.41 | 0.59 | 1352 | 2.96 | 1.81 | 0.59 | 125 | 3.76 | 1.88 | 0.50 |
| 2O8V | - | - | - | - | - | - | - | - | - | - | - | - |
| 2PCC | 920 | 6.29 | 2.19 | 0.44 | 975 | 5.67 | 2.17 | 0.39 | 458 | 6.29 | 2.19 | 0.44 |
| 2SIC | 1 | 6.47 | 1.44 | 0.52 | 3 | 8.89 | 2.40 | 0.43 | 3 | 5.92 | 1.19 | 0.73 |
| **2SNI** | **178** | **4.14** | **1.44** | **0.53** | **230** | **6.34** | **2.16** | **0.42** | **8** | **8.57** | **2.45** | **0.57** |
| 2UUY | 22 | 12.46 | 2.11 | 0.74 | 380 | 13.94 | 2.39 | 0.66 | 233 | 13.72 | 2.38 | 0.66 |
| **7CEI** | **3** | **4.33** | **1.36** | **0.65** | **1** | **0.68** | **2.44** | **0.44** | **4** | **8.41** | **2.46** | **0.65** |
| 1A2K | - | - | - | - | - | - | - | - | - | - | - | - |
| 1AK4 | - | - | - | - | - | - | - | - | - | - | - | - |
| 1AKJ | 175 | 6.35 | 2.15 | 0.53 | 637 | 4.22 | 1.70 | 0.65 | 395 | 3.30 | 1.54 | 0.60 |
| 1AZS | 123 | 11.64 | 1.86 | 0.69 | 8 | 2.49 | 1.74 | 0.65 | 1 | 11.04 | 1.88 | 0.69 |
| 1B6C | 2 | 7.35 | 2.37 | 0.74 | 1 | 3.47 | 2.38 | 0.87 | 2 | 3.67 | 2.23 | 0.90 |
| 1BUH | 353 | 4.13 | 1.56 | 0.60 | 18 | 3.42 | 1.81 | 0.63 | 1 | 3.42 | 1.81 | 0.63 |
| **1E96** | **24** | **5.56** | **1.99** | **0.54** | **219** | **9.54** | **2.38** | **0.42** | **261** | **6.03** | **2.18** | **0.58** |
| 1EFN | - | - | - | - | - | - | - | - | - | - | - | - |
| 1F51 | 237 | 3.41 | 1.82 | 0.60 | 368 | 3.41 | 1.82 | 0.60 | 16 | 3.41 | 1.82 | 0.60 |
| **1FC2** | **-** | **-** | **-** | **-** | **-** | **-** | **-** | **-** | **-** | **-** | **-** | **-** |
| 1FQJ | - | - | - | - | - | - | - | - | - | - | - | - |
| 1GCQ | 922 | 1.98 | 1.19 | 0.71 | 1171 | 1.98 | 1.19 | 0.71 | 118 | 1.98 | 1.19 | 0.71 |
| 1GHQ | - | - | - | - | - | - | - | - | - | - | - | - |
| 1GLA | 12 | 3.38 | 1.52 | 0.32 | 919 | 3.38 | 1.52 | 0.32 | 57 | 4.91 | 2.22 | 0.37 |
| 1GPW | 1 | 5.05 | 2.03 | 0.58 | 39 | 7.13 | 2.39 | 0.50 | 1 | 7.10 | 2.44 | 0.39 |
| 1HE1 | 43 | 6.92 | 2.20 | 0.47 | 2 | 8.68 | 2.35 | 0.47 | 301 | 5.93 | 1.74 | 0.38 |

(continued)

| Complex | ZDock | | | | ZRank | | | | ConvexPP | | | |
|---------|-------|------|------|------|-------|------|------|------|----------|------|------|------|
| | Rank | $L_{RMSD}$ | $I_{RMSD}$ | $f_{nat}$ | Rank | $L_{RMSD}$ | $I_{RMSD}$ | $f_{nat}$ | Rank | $L_{RMSD}$ | $I_{RMSD}$ | $f_{nat}$ |
| 1I4D | - | - | - | - | - | - | - | - | - | - | - | - |
| **1J2J** | **1897** | **5.59** | **2.12** | **0.55** | **277** | **5.59** | **2.12** | **0.55** | **86** | **5.59** | **2.12** | **0.55** |
| 1K74 | 1 | 5.86 | 2.29 | 0.42 | 1 | 6.11 | 2.35 | 0.52 | 8 | 7.90 | 2.02 | 0.48 |
| 1KAC | 11 | 4.47 | 2.07 | 0.40 | 438 | 5.35 | 2.22 | 0.36 | 287 | 4.47 | 2.21 | 0.36 |
| 1KLU | - | - | - | - | - | - | - | - | - | - | - | - |
| 1KTZ | 397 | 6.04 | 1.15 | 0.63 | 1486 | 4.25 | 1.54 | 0.74 | 282 | 5.39 | 1.25 | 0.63 |
| **1KXP** | **40** | **4.94** | **1.79** | **0.49** | **1** | **6.29** | **2.09** | **0.46** | **1** | **7.43** | **2.17** | **0.51** |
| 1ML0 | 1 | 2.27 | 1.25 | 0.76 | 1 | 5.05 | 2.07 | 0.51 | 1 | 4.47 | 1.89 | 0.61 |
| 1QA9 | - | - | - | - | - | - | - | - | - | - | - | - |
| 1RLB | 25 | 8.50 | 2.05 | 0.55 | 2 | 9.11 | 1.93 | 0.66 | 7 | 9.11 | 1.93 | 0.66 |
| 1S1Q | 1195 | 2.35 | 1.53 | 0.58 | 420 | 2.35 | 1.53 | 0.58 | 766 | 2.35 | 1.53 | 0.58 |
| 1SBB | - | - | - | - | - | - | - | - | - | - | - | - |
| 1T6B | 351 | 6.12 | 1.49 | 0.52 | 166 | 5.90 | 1.87 | 0.48 | 59 | 5.91 | 2.03 | 0.64 |
| 1XD3 | 135 | 6.68 | 2.21 | 0.55 | 62 | 4.87 | 2.49 | 0.30 | 6 | 4.87 | 2.49 | 0.30 |
| 1Z0K | 19 | 4.60 | 1.99 | 0.74 | 1 | 5.52 | 1.99 | 0.74 | 11 | 4.59 | 1.68 | 0.56 |
| 1Z5Y | 77 | 5.69 | 1.97 | 0.50 | 46 | 5.80 | 2.27 | 0.41 | 8 | 6.58 | 1.97 | 0.50 |
| **1ZHI** | **71** | **4.80** | **1.32** | **0.71** | **119** | **8.39** | **1.79** | **0.65** | **78** | **9.90** | **1.96** | **0.61** |
| 2AJF | - | - | - | - | - | - | - | - | - | - | - | - |
| 2BTF | 655 | 5.61 | 2.31 | 0.31 | 398 | 5.61 | 2.31 | 0.31 | 655 | 6.00 | 2.20 | 0.33 |
| 2HLE | 1 | 3.38 | 2.11 | 0.42 | 9 | 5.95 | 2.43 | 0.42 | 9 | 6.84 | 2.35 | 0.35 |
| 2HQS | 1092 | 8.94 | 2.30 | 0.37 | 1162 | 8.94 | 2.30 | 0.37 | 576 | 8.94 | 2.30 | 0.37 |
| 2O0B | - | - | - | - | - | - | - | - | - | - | - | - |
| Medium | | | | | | | | | | | | |
| 1BGX | - | - | - | - | - | - | - | - | - | - | - | - |
| **1ACB** | **-** | **-** | **-** | **-** | **-** | **-** | **-** | **-** | **-** | **-** | **-** | **-** |
| 1IJK | 444 | 7.43 | 1.65 | 0.31 | 376 | 5.02 | 1.35 | 0.38 | 124 | 6.42 | 1.83 | 0.25 |
| 1KKL | 1002 | 6.23 | 2.50 | 0.44 | 1774 | 6.23 | 2.50 | 0.44 | 325 | 6.23 | 2.50 | 0.44 |
| 1M10 | - | - | - | - | - | - | - | - | - | - | - | - |
| **1NW9** | **-** | **-** | **-** | **-** | **-** | **-** | **-** | **-** | **-** | **-** | **-** | **-** |
| 1GP2 | - | - | - | - | - | - | - | - | - | - | - | - |
| **1GRN** | **1365** | **7.10** | **2.49** | **0.00** | **676** | **7.10** | **2.49** | **0.00** | **1785** | **7.10** | **2.49** | **0.00** |
| 1HE8 | - | - | - | - | - | - | - | - | - | - | - | - |
| 1I2M | - | - | - | - | - | - | - | - | - | - | - | - |
| 1IB1 | - | - | - | - | - | - | - | - | - | - | - | - |
| 1K5D | 1111 | 8.04 | 2.03 | 0.29 | 466 | 8.04 | 2.03 | 0.29 | 1185 | 8.04 | 2.03 | 0.29 |
| 1N2C | - | - | - | - | - | - | - | - | - | - | - | - |
| 1WQ1 | - | - | - | - | - | - | - | - | - | - | - | - |

(continued)

| Complex | ZDock | | | | ZRank | | | | ConvexPP | | | |
|---|---|---|---|---|---|---|---|---|---|---|---|---|
| | Rank | $L_{RMSD}$ | $I_{RMSD}$ | $f_{nat}$ | Rank | $L_{RMSD}$ | $I_{RMSD}$ | $f_{nat}$ | Rank | $L_{RMSD}$ | $I_{RMSD}$ | $f_{nat}$ |
| 1XQS | 314 | 6.91 | 2.47 | 0.34 | 19 | 6.91 | 2.47 | 0.34 | 199 | 5.60 | 2.28 | 0.38 |
| 2CFH | 237 | 5.20 | 2.12 | 0.36 | 1 | 3.83 | 1.86 | 0.47 | 1 | 5.20 | 2.12 | 0.36 |
| 2H7V | 525 | 13.69 | 2.47 | 0.44 | 98 | 3.69 | 2.47 | 0.44 | 8 | 13.69 | 2.47 | 0.44 |
| 2HRK | - | - | - | - | - | - | - | - | - | - | - | - |
| 2NZ8 | - | - | - | - | - | - | - | - | - | - | - | - |
| Difficult | | | | | | | | | | | | |
| 1E4K | - | - | - | - | - | - | - | - | - | - | - | - |
| 2HMI | - | - | - | - | - | - | - | - | - | - | - | - |
| 1FQ1 | - | - | - | - | - | - | - | - | - | - | - | - |
| 1PXV | - | - | - | - | - | - | - | - | - | - | - | - |
| 1ATN | - | - | - | - | - | - | - | - | - | - | - | - |
| 1BKD | - | - | - | - | - | - | - | - | - | - | - | - |
| 1DE4 | - | - | - | - | - | - | - | - | - | - | - | - |
| 1EER | - | - | - | - | - | - | - | - | - | - | - | - |
| 1FAK | - | - | - | - | - | - | - | - | - | - | - | - |
| 1H1V | - | - | - | - | - | - | - | - | - | - | - | - |
| 11BR | - | - | - | - | - | - | - | - | - | - | - | - |
| 1IRA | - | - | - | - | - | - | - | - | - | - | - | - |
| 1JMO | - | - | - | - | - | - | - | - | - | - | - | - |
| 1R8S | - | - | - | - | - | - | - | - | - | - | - | - |
| 1Y64 | - | - | - | - | - | - | - | - | - | - | - | - |
| 2C0L | - | - | - | - | - | - | - | - | - | - | - | - |
| 2OT3 | - | - | - | - | - | - | - | - | - | - | - | - |
| Homologs included | Top1: 8.1% (10/124) Top10: 16.1% (20/124) | | | | Top1: 12.9% (16/124) Top10: 22.6% (28/124) | | | | Top1: 10.5% (13/124) Top10: 27.4% (34/124) | | | |
| Homologs excluded | | | | | | | | | Top1:12.1% (15/124) Top10: 29.0% (36/124) | | | |

Figure 13 shows ROC curves (success rate versus the number of top predictions considered). One may see that ConvexPP scoring functions outperform ZRANK and ZDOCK if the number of considered predictions is more than eight.

### 12.2 - Rosetta Benchmark

Baker, Gray *et al* generated the Rosetta benchmark using 54 complexes of the protein-protein docking benchmark version 0.0[18] using a flexible docking protocol, which is a part of the RosettaDock suite[19]. The first step in the protocol is the random translation and rotation of one of the proteins constituting the complex. Afterwards, the side chain is optimized simultaneously with the rigid body displacement. Finally, the full-atom minimization is done to refine the conformation. For each complex, Baker and Gray generated 1000 decoys following the described protocol. The success rate of RosettaDock was calculated using the same quality criteria as in Critical Assessment of PRediction of Interactions[20]. The Rosetta benchmark contains 5 complexes homologous to the ones present in the training set. Therefore the scoring function according to the invention was trained using training sets with and without these homologs. Table 6 compares the results of RosettaDock[19], ITScore-PP[13] and our ConvexPP scoring functions.

Table 6 shows that the potentials according to the invention significantly improve Top1 prediction rate over ITScore-PP and RosettaDock scoring functions while also outperforming them according to the other criteria (Top1 and quality 1 *etc.*). The percentage of the structures for which the first acceptable prediction was ranked within the top predictions was computed for each complex and plotted on Fig. 14. According to the plot, the scoring function of the invention (ConvexPP) outputs the plausible structure (quality $\geq$3) for more complexes than ITScore-PP and RosettaDock. Unlike the results on the ZDock benchmark, the results on the Rosetta unbound benchmark slightly decrease when homologous complexes were removed from the training set. Among the prediction quality criteria it is the number of predicted high quality structures that changed the most. On the other hand Top1 prediction rate stayed almost the same. This observation means that the number of predicted high-quality structures is amenable to overfitting. Therefore unlike the Top1 criterion, it can not serve as a reliable measure of a scoring function predictive power.

Table 6: Rosetta unbound benchmark results. Proteins homologous to the ones in the training set are shown in bold. The $L_{RMSD}$ parameter represents the quality of a pose, which is the RMSD of the backbone atoms of the ligand after the receptors in the native and the decoy conformations have been optimally superimposed. The $I_{RMSD}$ parameter is the RMSD of the interface region between the predicted and native structures after optimal superimposition of the backbone atoms of the interface residues. A residue is considered as the interface residue if any atom of this residue is within 10 A from the other partner. The $f_{nat}$ parameter is the ratio of the number of native residue-residue contacts in the predicted complex to the number of residue-residue contacts in the crystal structure. To assign the quality for the docking predictions, we use the criterion from Critical Assessment of PRediction of Interactions (CAPRI).

Table 6: Rosetta unbound benchmark results

| Complex | Rosetta Dock | | | | | ITScorePP | | | | | ConvexPP | | | | |
|---|---|---|---|---|---|---|---|---|---|---|---|---|---|---|---|
| | Quality | Rank | $L_{RMSD}$ | $I_{RMSD}$ | $f_{nat}$ | Quality | Rank | $L_{RMSD}$ | $I_{RMSD}$ | $f_{nat}$ | Quality | Rank | $L_{RMSD}$ | $I_{RMSD}$ | $f_{nat}$ |
| 1ACB | 3 | 3 | 3.41 | 9.08 | 0.13 | 3 | 4 | 3.39 | 9.04 | 0.11 | 2 | 1 | 1.13 | 4.31 | 0.778 |
| 1A0O | 2 | 1 | 4.18 | 10.29 | 0.57 | 2 | 1 | 4.18 | 10.29 | 0.57 | 3 | 1 | 5.79 | 11.3 | 0.353 |
| 1AHW | 2 | 1 | 2.37 | 6.41 | 0.51 | 3 | 4 | 3.00 | 6.46 | 0.49 | 2 | 1 | 2.31 | 4.82 | 0.44 |
| 1ATN | 3 | 1 | 2.79 | 5.83 | 0.49 | 3 | 1 | 4.70 | 9.49 | 0.38 | 1 | 1 | 0.708 | 2.34 | 0.765 |
| 1AVW | 2 | 1 | 2.09 | 6.02 | 0.67 | 2 | 1 | 1.81 | 5.07 | 0.71 | 2 | 1 | 1.38 | 6.01 | 0.746 |
| 1AVZ | 3 | 37 | 4.05 | 8.08 | 0.29 | 3 | 22 | 5.55 | 11.47 | 0.35 | 3 | 20 | 4.41 | 8.41 | 0.122 |
| 1BQL | 1 | 1 | 0.86 | 1.57 | 0.64 | 1 | 5 | 0.72 | 1.81 | 0.65 | 1 | 1 | 0.96 | 1.4 | 0.895 |
| 1BRC | 2 | 4 | 1.21 | 3.77 | 0.75 | 2 | 1 | 1.21 | 3.77 | 0.75 | 2 | 1 | 1.62 | 7.24 | 0.759 |
| 1BRS | 2 | 1 | 1.73 | 4.78 | 0.64 | 3 | 1 | 4.46 | 8.68 | 0.33 | 3 | 1 | 3.79 | 8.7 | 0.309 |
| 1BTH | 3 | 4 | 5.54 | 18.21 | 0.30 | 3 | 2 | 2.35 | 5.60 | 0.42 | 3 | 1 | 2.67 | 5.59 | 0.44 |
| 1BVK | 3 | 1 | 3.93 | 7.91 | 0.20 | 3 | 1 | 3.54 | 7.18 | 0.20 | 3 | 1 | 3.45 | 7.75 | 0.222 |
| 1CGI | 2 | 2 | 1.86 | 3.79 | 0.50 | 3 | 8 | 2.37 | 6.01 | 0.42 | 3 | 3 | 2.11 | 6.44 | 0.376 |
| 1CHO | 3 | 1 | 2.19 | 6.31 | 0.46 | 3 | 1 | 3.76 | 10.32 | 0.18 | 2 | 1 | 1.81 | 6.35 | 0.661 |
| 1CSE | 2 | 6 | 3.12 | 10.10 | 0.56 | 2 | 1 | 2.66 | 8.81 | 0.71 | 3 | 1 | 2.29 | 7.87 | 0.4 |
| 1DFJ | 2 | 1 | 2.66 | 5.69 | 0.59 | 2 | 1 | 2.66 | 5.69 | 0.59 | 2 | 1 | 2.55 | 5.63 | 0.671 |
| 1DQJ | 3 | 1 | 3.35 | 6.71 | 0.31 | 3 | 5 | 2.12 | 5.00 | 0.34 | 3 | 1 | 6.06 | 14 | 0.379 |
| 1EFU | 3 | 44 | 3.78 | 5.98 | 0.16 | 3 | 26 | 4.21 | 7.83 | 0.10 | 3 | 20 | 4.65 | 5.9 | 0.106 |
| 1EO8 | 3 | 1 | 5.54 | 10.73 | 0.31 | 3 | 31 | 3.36 | 6.12 | 0.15 | 3 | 1 | 3.29 | 10.9 | 0.42 |
| 1FBI | 2 | 1 | 1.37 | 2.79 | 0.54 | 2 | 1 | 1.86 | 3.64 | 0.51 | 3 | 1 | 4.23 | 11 | 0.357 |
| 1FIN | 3 | 364 | 5.38 | 9.88 | 0.12 | 3 | 109 | 4.19 | 8.26 | 0.12 | 3 | 200 | 6.06 | 8.27 | 0.102 |
| 1FQ1 | 3 | 1 | 5.43 | 11.37 | 0.31 | 3 | 1 | 5.09 | 9.33 | 0.41 | 3 | 1 | 4.02 | 6.79 | 0.434 |
| 1FSS | 1 | 1 | 0.97 | 3.07 | 0.74 | 2 | 1 | 1.42 | 4.46 | 0.46 | 2 | 1 | 1.54 | 3.89 | 0.631 |
| 1GLA | 2 | 4 | 1.85 | 5.96 | 0.65 | 3 | 7 | 3.83 | 11.96 | 0.35 | 2 | 1 | 1.7 | 6.2 | 0.842 |
| 1GOT | 3 | 12 | 3.95 | 7.86 | 0.19 | 3 | 4 | 4.26 | 9.71 | 0.19 | 3 | 2 | 3.21 | 12.9 | 0.173 |
| 1IAI | 3 | 8 | 3.42 | 6.60 | 0.22 | 2 | 1 | 1.61 | 4.18 | 0.62 | 2 | 1 | 1.62 | 3.82 | 0.333 |
| 1IGC | 1 | 1 | 0.63 | 2.15 | 0.85 | 1 | 1 | 0.63 | 2.15 | 0.85 | 1 | 1 | 0.561 | 1.92 | 1 |
| 1JHL | 3 | 3 | 4.51 | 8.56 | 0.26 | 2 | 2 | 2.66 | 6.09 | 0.56 | 3 | 2 | 3.94 | 7.65 | 0.308 |
| 1MAH | 2 | 1 | 1.19 | 3.72 | 0.70 | 3 | 1 | 2.77 | 8.66 | 0.26 | 2 | 1 | 1.15 | 3.71 | 0.778 |
| 1MDA | 3 | 1 | 3.59 | 8.77 | 0.19 | 3 | 1 | 3.92 | 9.84 | 0.26 | 2 | 2 | 2.38 | 6.65 | 0.524 |
| 1MEL | 2 | 1 | 2.62 | 8.47 | 0.50 | 2 | 4 | 2.62 | 8.47 | 0.50 | 2 | 1 | 2.84 | 7.89 | 0.516 |

| | | | | | | | | | | | | | | | |
|---|---|---|---|---|---|---|---|---|---|---|---|---|---|---|---|
| 1MLC | 2 | 7 | 1.37 | 4.91 | 0.52 | 3 | 1 | 3.45 | 15.36 | 0.20 | 3 | 1 | 3.04 | 15.4 | 0.238 |
| 1NCA | 2 | 1 | 1.53 | 3.06 | 0.61 | 1 | 1 | 0.64 | 1.24 | 0.75 | 2 | 1 | 2.13 | 7.62 | 0.659 |
| 1NMB | 1 | 1 | 0.44 | 0.90 | 0.80 | 1 | 6 | 0.76 | 2.66 | 0.85 | 1 | 1 | 0.569 | 2.66 | 1 |
| 1PPE | 1 | 1 | 0.52 | 1.38 | 0.73 | 3 | 1 | 2.39 | 7.42 | 0.28 | 1 | 1 | 0.54 | 1.38 | 0.887 |
| 1QFU | 2 | 1 | 1.10 | 3.02 | 0.64 | 1 | 4 | 1.00 | 2.93 | 0.69 | 1 | 1 | 0.982 | 3.89 | 0.673 |
| 1SPB | 1 | 1 | 0.62 | 1.06 | 0.68 | 1 | 1 | 0.70 | 1.47 | 0.69 | 1 | 1 | 0.538 | 1.04 | 0.819 |
| 1STF | 1 | 1 | 0.68 | 1.91 | 0.89 | 1 | 2 | 0.54 | 1.57 | 0.91 | 1 | 1 | 0.514 | 1.57 | 0.944 |
| 1TAB | 2 | 1 | 1.30 | 4.16 | 0.74 | 2 | 1 | 1.37 | 4.39 | 0.76 | 2 | 1 | 1.47 | 4.11 | 0.677 |
| 1TGS | 2 | 1 | 1.44 | 2.48 | 0.59 | 2 | 1 | 1.38 | 2.26 | 0.64 | 3 | 1 | 3.55 | 8.41 | 0.438 |
| 1UDI | 2 | 1 | 1.43 | 3.35 | 0.63 | 2 | 1 | 1.01 | 2.08 | 0.74 | 2 | 1 | 1.09 | 2.08 | 0.707 |
| 1UGH | 1 | 1 | 0.86 | 1.78 | 0.67 | 2 | 1 | 1.90 | 4.64 | 0.46 | 2 | 1 | 1.96 | 4.61 | 0.598 |
| 1WEJ | 3 | 15 | 2.92 | 9.28 | 0.44 | 2 | 2 | 2.55 | 6.90 | 0.62 | 3 | 1 | 4.79 | 9.37 | 0.227 |
| 1WQ1 | 3 | 1 | 2.46 | 5.53 | 0.34 | 2 | 1 | 1.92 | 3.38 | 0.39 | 2 | 2 | 1.59 | 3.83 | 0.582 |
| 2BTF | 3 | 1 | 3.23 | 10.03 | 0.22 | 1 | 1 | 0.60 | 1.52 | 0.75 | 1 | 1 | 0.598 | 1.31 | 0.897 |
| 2JEL | 2 | 1 | 2.22 | 4.82 | 0.52 | 2 | 1 | 1.98 | 6.55 | 0.65 | 2 | 1 | 1.26 | 6.42 | 0.857 |
| 2KAI | 1 | 2 | 0.97 | 2.02 | 0.67 | 2 | 2 | 1.05 | 2.48 | 0.70 | 1 | 8 | 0.892 | 2.26 | 0.882 |
| 2PCC | 3 | 2 | 3.39 | 9.19 | 0.24 | 3 | 1 | 3.84 | 9.38 | 0.29 | 3 | 1 | 4.31 | 9.4 | 0.483 |
| 2PTC | 1 | 4 | 0.44 | 0.82 | 0.80 | 2 | 4 | 1.82 | 5.86 | 0.70 | 2 | 1 | 1.16 | 5.3 | 0.735 |
| 2SIC | 2 | 1 | 1.53 | 5.18 | 0.85 | 2 | 1 | 1.53 | 5.18 | 0.85 | 1 | 1 | 0.956 | 4.84 | 0.817 |
| 2SNI | 2 | 1 | 2.01 | 6.70 | 0.60 | 1 | 1 | 0.99 | 2.88 | 0.73 | 2 | 1 | 2 | 7.38 | 0.606 |
| 2TEC | 1 | 1 | 0.81 | 2.46 | 0.74 | 1 | 1 | 0.85 | 2.59 | 0.78 | 1 | 1 | 0.591 | 1.97 | 0.8 |
| 2VIR | 3 | 1 | 4.19 | 7.53 | 0.26 | 2 | 2 | 2.17 | 5.74 | 0.70 | 2 | 1 | 1.03 | 5.78 | 0.673 |
| 3HHR | 3 | 50 | 3.95 | 9.84 | 0.26 | 3 | 3 | 4.03 | 8.17 | 0.30 | 3 | 1 | 3.47 | 9.41 | 0.329 |
| 4HTC | 2 | 1 | 1.54 | 3.81 | 0.61 | 3 | 1 | 2.25 | 5.95 | 0.42 | 2 | 1 | 1.5 | 4.04 | 0.76 |
| | Top1: 66.7% (36/54)<br><br>Rank 1 and Quality 1: 14.8% (8/54)<br><br>Rank 1 and Quality 1 ‖ 2: 46.2% (25/54)<br><br>Rank<10 and Quality 1 ‖ 2: 61.1% (33/54) | | | | | | Top1: 59.3% (32/54)<br><br>Rank 1 and Quality 1: 11.1% (6/54)<br><br>Rank 1 and Quality 1 ‖ 2: 42.6% (23/54)<br><br>Rank<10 and Quality 1 ‖ 2: 57.4% (31/54) | | | | | | Top1: 83.3% (45/54)<br><br>Rank 1 and Quality 1: 20.4% (11/54)<br><br>Rank 1 and Quality 1 ‖ 2: 57.4% (31/54)<br><br>Rank<10 and Quality 1 ‖ 2: 63.0% (34/54) | | | | |

Bibliography:

1. Cheng, T., Li, X., Li, Y., Liu, Z., & Wang, R. 2009, Journal of Chemical Information and Modeling, 49, 1079-1093.
2. Rarey, M., Kramer, B., Lengauer, T., & Klebe, G. 1996, Journal of Molecular Biology, 261, 470-489.
3. Tanaka, S. & Scheraga, H. A. 1976, Macromolecules, 9, 945-950.
4. Miyazawa, S. & Jernigan, R. L. 1985, Macromolecules, 18, 534-552.
5. Sippl, M. J. 1990, Journal of Molecular Biology, 213, 859-883.
6. Clark, M., Cramer, R. D., & Van Opdenbosch, N. 1989, Journal of Computational Chemistry, 10, 982-1012.
7. Neudert, G. & Klebe, G. 2011, Bioinformatics (Oxford, England), 27, 1021.
8. Ritchie, D. W. & Kemp, G. J. L. 2000, Proteins: Structure, Function, and Bioinformatics, 39, 178-194.
9. OLBoyle, N. M., Banck, M., James, C. A., Morley, C., Vandermeersch, T., & Hutchison, G. R. 2011, Journal of Cheminformatics, 3, 33.
10. Vapnik, V. 1999, The nature of statistical learning theory.
11. Cortes, C. & Vapnik, V. 1995, Machine Learning, 20, 273-297.
12. Burges, C. J. C. & Crisp, D. J. 2000, in Advances in Neural Information Processing Systems, Vol. 12, 223-229.
13. Huang, S. Y. & Zou, X. 2008, Proteins: Structure, Function, and Bioinformatics, 72, 557-579.
14. Wang, R., Fang, X., Lu, Y., & Wang, S. 2004, Journal of Medicinal Chemistry, 47, 2977-2980.

15. Berman, H. M., Westbrook, J., Feng, Z., Gilliland, G., Bhat, T., Weissig, H., Shindyalov, I. N., & Bourne, P. E. 2000, Nucleic Acids Research, 28, 235-242.

16. Neudert, G. & Klebe, G. 2011, Journal of Chemical Information and Modeling, 51, 2731-45.

17. Hwang, H., Pierce, B., Mintseris, J., Janin, J., & Weng, Z. 2008, Proteins: Structure, Function, and Bioinformatics, 73, 705-709.

18. Chen, R. & Weng, Z. 2002, Proteins: Structure, Function, and Bioinformatics, 47, 281-294.

19. Gray, J. J., Moughon, S., Wang, C., Schueler-Furman, O., Kuhlman, B., Rohl, C. A., & Baker, D. 2003, Journal of Molecular Biology, 331, 281-300.

20. Méndez, R., Leplae, R., De Maria, L., & Wodak, S. J. 2003, Proteins: Structure, Function, and Bioinformatics, 52, 51-67.

## Claims

1. A method for modeling the geometric structure of the interface of Receptor-Ligand complexes, wherein a first chemical molecule defined as Receptor and a second chemical molecule defined as Ligand, wherein said method comprises the following steps wherein at least one of them is implemented or assisted by computer:

   (a) providing a set of Receptors and Ligands from at least one computer database, wherein Receptor-Ligand complexes present an interface comprising different atom types, wherein atom type k is located on the Receptor and atom type I is located on the Ligand interact, k and I varying depending on the atom type;
   (b) selecting atoms from at the Receptor-Ligand complexes interface of said Receptors and Ligands;
   (c) assigning to each selected atoms an atom type among k and I;
   (d) providing for Receptor-Ligand complexes the distances $r_{ij}$ between an atom i of a specific atom type k of the Receptor and an atom j of a specific atom type I of the Ligand, wherein i index runs over specific atoms among atom type k, and wherein j index runs over specific atoms among atom type I ;
   (e) optionally repeating step (c) for all or other atoms types k and I;
   (f) assigning the distances $r_{ij}$ as a function of atom types; and
   (g) providing the modeling of the geometric structure of the interface of Receptor-Ligand complexes as a function of distances $r_{ij}$.

2. The method of claim 1, wherein said modeling of the geometric structure of the interface of Receptor-Ligand complexes takes into account inaccuracies in the determination of distances $r_{ij}$.

3. The method of claim 1, wherein the modeling of the geometric structure of the interface of Receptor-Ligand complexes as a function of distances $r_{ij}$. is defined by the number densities $n^{kl}(r)$, wherein said number densities $n^{kl}(r)$ is defined as:

$$n^{kl}(r) = \sum_{ij} \frac{1}{\sqrt{2\pi\sigma^2}} e^{-\frac{(r-r_{ij})^2}{2\sigma^2}}, \qquad (4)$$

wherein each distance distribution is represented by a Gaussian centered at $r_{ij}$ with the constant variance of $\sigma^2$, and wherein the distance $r_{ij}$ is smaller than a determined cutoff distance $r_{max}$.

4. A method for generating virtual Non-Native Receptor-Ligand complexes, wherein said method comprises the following steps wherein at least one of them is implemented or assisted by computer:

   (a) providing a set of Native Receptor-Ligand complexes $P_i^{nat}$ from at least one computer database, wherein Receptor-Ligand complexes present an interface wherein site k of the Receptor and site I of the Ligand interact;

   (b) generating D Non-Native Receptor-Ligand complexes $P_j^{nonnat}$, j = 1... D wherein j index runs over generated decoys and D represents the total number of Non-Native Receptor-Ligand complexes generated, wherein Non-Native Receptor-Ligand complexes are generated by moving spatially the Ligand relative to the Receptor or by local deformation along spatial directions from the Native Receptor-Ligand complexes.

**5.** The method of claim 4, wherein Non-Native Receptor-Ligand complexes $P_j^{nonnat}$, are generated by rolling the Ligand over the surface of the Receptor.

**6.** The method of claim 4, wherein Non-Native Receptor-Ligand complexes $P_j^{nonnat}$, are generated by the following steps:

- Considering a Ligand as a rigid body,
- Rotating the Ligand about one or more rotational axes, and
- Translating the Ligand along the coordinate axes.

**7.** The method of claim 6, wherein Non-Native Receptor-Ligand complexes $P_j^{nonnat}$, are generated by linear combinations of modes $\{v_i\}$ as follows::

$$\mathbf{X}^{decoy} = \mathbf{X}^{native} + \sqrt{k_B \mathbf{T}} \sum_{i=6}^{15} r_i \frac{v_i}{\omega_i},$$

where $\mathbf{X}^{native}$ and $\mathbf{X}^{decoy}$ are the coordinate vectors corresponding to the native and non-native conformations, respectively, $r_i$ is the random weight for each mode ranging from -1 to 1, and $\omega_i$ is the frequency of the mode $v_i$.

**8.** A method for modeling the interaction between a Receptor and a Ligand in Receptor-Ligand complexes, wherein said method comprises the following steps wherein at least one of them is implemented or assisted by computer:

(a) providing a set of Receptors and Ligands from at least one computer database;
(b) assigning to each Receptor-Ligand complex a specific structure vector x which is a mathematical vector representing the specific geometric structure of the interface between a Receptor and a Ligand in a specific Receptor-Ligand complex;
(c) computing a linear convex scoring function F as a function of all specific structure vectors x or of vector X which is the concatenation of all vectors x thereof, preferably said linear convex scoring function F being also a function of a scoring vector w;
(d) projecting said scoring function F in orthogonal polynomial subspaces; thereby modeling the interaction between a Receptor and a Ligand in Receptor-Ligand complexes.

**9.** A method for determining a scoring vector *w* which is a mathematical vector quantifying and/or qualifying the interaction of a geometric structure of the interface of a Receptor-Ligand complex, wherein Receptor-Ligand complexes present an interface in interaction, wherein said interaction is in need for quantification and/or qualification, wherein said method comprises the following steps wherein at least one of them is implemented or assisted by computer:

(a) providing a set of Receptors and Ligands from at least one computer database;
(b) assigning to each geometric structure of an interface of a Receptor-Ligand complex, a specific structure vector *x* which is a mathematical vector representing the specific geometric structure of the interface;
(c) computing a linear convex scoring function F as a function of all specific structure vectors x and scoring vector w;
(e) projecting said scoring function F in orthogonal polynomial subspaces;
(f) formulating a convex optimization problem;
(g) solving the convex optimization problem thereby determining a scoring vector w.

**10.** The method of claim 9, wherein said step (a) comprises providing Native Receptor-Ligand complex $P_i^{nat}$ and Non-native Receptor-Ligand complexes $P_{ij}^{nonnat}$, wherein *i* index runs over different protein complexes.

**11.** The method of claim 9, wherein said step (b) comprises implementing a method as defined by any one of claims 1 to 3.

**12.** The method of claim 9, wherein in step (e) orthogonal polynomial subspaces are Rectangular, Legendre, Laguerre or Fourier orthogonal bases.

13. The method of claim 9, wherein step (f) comprises using the artificially generated noise applied to the original input data wherein said noise is represented by the Gaussian distance distribution of the input data having a variance $\sigma$ where $\sigma$ is constant and does not depend on the atom type, and can be thought as a Gaussian filter applied to the input data if the latter is represented as a 1 D signal.

14. The method of claim 9, wherein step (f) comprises formulating a convex optimization problem so as to minimize the convex function.

15. The method of any one of claims 9 to 15, wherein said method further comprises finding the scoring vector w.

16. A method for determining the binding affinity or binding free energy of a position of a Ligand relative to a Receptor in one or more Receptor-Ligand complexes, wherein said Receptor-Ligand complex presents an interface comprising different atom types, wherein atom type k, located on the Receptor, and atom type l, located on the Ligand, interact, k and l varying depending on the atom type, wherein said method comprises the following steps wherein at least one of them is implemented or assisted by computer:

   (i) modeling the geometric structure of the interface of one or more Receptor-Ligand complexes, said modeling being as defined in any one of claims 1 to 7
   (ii) assigning to a geometric structure of the interface of Receptor-Ligand complex, a binding affinity or binding free energy by reference to a database, optionally wherein said binding affinity or binding free energy is determined as a scalar product of the structure vector x with the scoring vector w as defined in the method of claim 9.

17. A method for ranking the binding affinity or binding free energy of spatial positions of a Ligand relative to a Receptor in one or more Receptor-Ligand complexes, wherein said method comprises the following steps wherein at least one of them is implemented or assisted by computer:

   (i) implementing the method of claim 16 to determine the binding affinity or binding free energy of two or more spatial positions of a Ligand relative to a Receptor in one or more Receptor-Ligand complexes,
   (ii) ranking spatial positions of a Ligand relative to a Receptor based on said binding affinity or binding free energy by providing a strict relationship between a set of spatial positions such that, for any two positions, the first is either ranked higher, lower or equal to the second position if the binding energy of the first position is smaller, equal or higher than the energy of the second position, respectively.

18. The method of claim 17, wherein the best spatial position of a Ligand relative to a Receptor in one or more Receptor-Ligand complexes is determined based on the ranking of said binding affinity or binding free energy.

19. The method of claim 17, wherein the best binding affinity or free binding energy among several Receptor-Ligand complexes is determined based on the ranking of said binding affinity or binding free energy.

FIG.1

FIG.2

```
                    ┌─────────┐
                    │   -1-   │
                    └─────────┘
                         │
                         ▼
            ┌──────────►┌─────────┐
            │           │   -2-   │
            │           └─────────┘
            │                │
            │                ▼
            │           ┌─────────┐
            │           │   -3-   │
            │           └─────────┘
            │                │
            │                ▼
            │           ┌─────────┐
            │           │   -4-   │
            │           └─────────┘
            │                │
            │                ▼
            │           ┌─────────┐
            │           │   -5-   │
            │           └─────────┘
            │          Receptor    Ligand
            │         ┌────────┐  ┌────────┐
            │         ▼         ▼
            │     ┌────────┐  ┌────────┐
            │     │ -6.1a- │  │ -6.1-  │
            │     └────────┘  └────────┘
            │         │           │
            │         ▼           ▼
            │     ┌────────┐  ┌────────┐
            │     │ -6.2a- │  │ -6.2-  │
            │     └────────┘  └────────┘
            │         │           │
            │         ▼           ▼
            │           ┌─────────┐
            │           │   -7-   │
            │           └─────────┘
            │                │
            │                ▼
            │           ┌─────────┐
            └───────────│   -8-   │
                        └─────────┘
                             │
                             ▼
                        ┌─────────┐
                        │   -9-   │
                        └─────────┘
                             │
                             ▼
                        ┌─────────┐
                        │  -10-   │
                        └─────────┘
```

FIG.3

FIG.4

EP 3 026 588 A1

A

B

FIG.5

FIG.6

FIG.7

A

C3C - C3C

B

N2+ - O2-

FIG.8

**FIG.9**

FIG.10

FIG.11

## FIG.12

## FIG.13

FIG.14

Europäisches
Patentamt

European
Patent Office

Office européen
des brevets

# EUROPEAN SEARCH REPORT

Application Number

EP 14 30 6882

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/16930 A2 (RIBOTARGETS LTD [GB]; AFSHAR MOHAMMAD MICHEL [GB]; MORLEY STEPHEN DAVI) 28 February 2002 (2002-02-28) * the whole document * | 1-3,11 | INV.<br>G06F19/00<br>G06F19/16 |
| X | WO 2006/099178 A2 (SCHRODINGER INC [US]; FRIESNER RICHARD A [US]; MURPHY ROBERT [US] SCHR) 21 September 2006 (2006-09-21) * the whole document * | 1-3,11 | |
| X | US 2009/006040 A1 (HRNCIAR PETER [US]) 1 January 2009 (2009-01-01) * the whole document * | 1-3,11 | |
| X | US 2013/166261 A1 (YAN ZHIQIANG [US] ET AL) 27 June 2013 (2013-06-27) * the whole document * | 1-3,11 | |
| X | SHERMAN W. ET AL: "Novel Procedure for Modeling Ligand/Receptor Induced Fit Effects", JOURNAL OF MEDICINAL CHEMISTRY, vol. 49, no. 2, 23 December 2005 (2005-12-23), pages 534-553, XP055186996, ISSN: 0022-2623, DOI: 10.1021/jm050540c * the whole document * | 1-3,11 | TECHNICAL FIELDS SEARCHED (IPC)<br><br>G06F |

~~The present search report has been drawn up for all claims~~

| Place of search | Date of completion of the search | Examiner |
|---|---|---|
| The Hague | 30 April 2015 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

Application Number

EP 14 30 6882

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-3(completely); 11(partially)

☐ The present supplementary European search report has been drawn up for those parts of the European patent application which relate to the invention first mentioned in the claims (Rule 164 (1) EPC).

**Europäisches Patentamt**
**European Patent Office**
**Office européen des brevets**

**LACK OF UNITY OF INVENTION**
**SHEET B**

Application Number

EP 14 30 6882

The Search Division considers that the present European patent application does not comply with the requirements of unity of invention and relates to several inventions or groups of inventions, namely:

1. claims: 1-3(completely); 11(partially)

   A method for modeling the geometric structure of the interface of Receptor-Ligand complexes.
   ---

2. claims: 4-7

   A method for generating virtual Non-Native Receptor-Ligand complexes.
   ---

3. claim: 8

   A method for modeling the interaction between a Receptor and a Ligand in Receptor-Ligand complexes.
   ---

4. claims: 9, 10, 12-15(completely); 11(partially)

   A method for determining a scoring vector w which is a mathematical vector quantifying and/or qualifying the interaction of a geometric structure of the interface of a Receptor-Ligand complex.
   ---

5. claims: 16-19

   A method for determining and ranking the binding affinity or binding free energy of a position of a Ligand relative to a Receptor in one or more Receptor-Ligand complexes.
   ---

**EP 3 026 588 A1**

## ANNEX TO THE EUROPEAN SEARCH REPORT
## ON EUROPEAN PATENT APPLICATION NO.

EP 14 30 6882

This annex lists the patent family members relating to the patent documents cited in  the above-mentioned European search report.
The members are as contained in the European Patent Office EDP file on
The European Patent Office is in no way liable for these particulars which are merely given for the purpose of information.

30-04-2015

| Patent document cited in search report | | Publication date | Patent family member(s) | | Publication date |
|---|---|---|---|---|---|
| WO 0216930 | A2 | 28-02-2002 | AU | 7997301 A | 04-03-2002 |
| | | | WO | 0216930 A2 | 28-02-2002 |
| WO 2006099178 | A2 | 21-09-2006 | CN | 101542284 A | 23-09-2009 |
| | | | DK | 1856244 T3 | 04-11-2013 |
| | | | EP | 1856244 A2 | 21-11-2007 |
| | | | ES | 2432753 T3 | 05-12-2013 |
| | | | JP | 4719223 B2 | 06-07-2011 |
| | | | JP | 2008518369 A | 29-05-2008 |
| | | | US | 2007061118 A1 | 15-03-2007 |
| | | | WO | 2006099178 A2 | 21-09-2006 |
| US 2009006040 | A1 | 01-01-2009 | US | 2008319677 A1 | 25-12-2008 |
| | | | US | 2009006040 A1 | 01-01-2009 |
| | | | US | 2009018777 A1 | 15-01-2009 |
| | | | WO | 2008144776 A1 | 27-11-2008 |
| US 2013166261 | A1 | 27-06-2013 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHENG, T. ; LI, X. ; LI, Y. ; LIU, Z. ; WANG, R.** *Journal of Chemical Information and Modeling,* 2009, vol. 49, 1079-1093
- **RAREY, M. ; KRAMER, B. ; LENGAUER, T. ; KLEBE, G.** *Journal of Molecular Biology,* 1996, vol. 261, 470-489
- **TANAKA, S. ; SCHERAGA, H. A.** *Macromolecules,* 1976, vol. 9, 945-950
- **MIYAZAWA, S. ; JERNIGAN, R. L.** *Macromolecules,* 1985, vol. 18, 534-552
- **SIPPL, M. J.** *Journal of Molecular Biology,* 1990, vol. 213, 859-883
- **CLARK, M. ; CRAMER, R. D. ; VAN OPDEN-BOSCH, N.** *Journal of Computational Chemistry,* 1989, vol. 10, 982-1012
- **NEUDERT, G. ; KLEBE, G.** *Bioinformatics (Oxford, England),* 2011, vol. 27, 1021
- **RITCHIE, D. W. ; KEMP, G. J. L.** *Proteins: Structure, Function, and Bioinformatics,* 2000, vol. 39, 178-194
- **OLBOYLE, N. M. ; BANCK, M. ; JAMES, C. A. ; MORLEY, C. ; VANDERMEERSCH, T. ; HUTCHISON, G. R.** *Journal of Cheminformatics,* 2011, vol. 3, 33
- **VAPNIK, V.** *The nature of statistical learning theory.,* 1999
- **CORTES, C. ; VAPNIK, V.** *Machine Learning,* 1995, vol. 20, 273-297

- **BURGES, C. J. C. ; CRISP, D. J.** *Advances in Neural Information Processing Systems,* 2000, vol. 12, 223-229
- **HUANG, S. Y. ; ZOU, X.** *Proteins: Structure, Function, and Bioinformatics,* 2008, vol. 72, 557-579
- **WANG, R. ; FANG, X. ; LU, Y. ; WANG, S.** *Journal of Medicinal Chemistry,* 2004, vol. 47, 2977-2980
- **BERMAN, H. M. ; WESTBROOK, J. ; FENG, Z. ; GILLILAND, G. ; BHAT, T. ; WEISSIG, H. ; SHINDY-ALOV, I. N. ; BOURNE, P. E.** *Nucleic Acids Research,* 2000, vol. 28, 235-242
- **NEUDERT, G. ; KLEBE, G.** *Journal of Chemical Information and Modeling,* 2011, vol. 51, 2731-45
- **HWANG, H. ; PIERCE, B. ; MINTSERIS, J. ; JANIN, J. ; WENG, Z.** *Proteins: Structure, Function, and Bioinformatics,* 2008, vol. 73, 705-709
- **CHEN, R. ; WENG, Z.** *Proteins: Structure, Function, and Bioinformatics,* 2002, vol. 47, 281-294
- **GRAY, J. J. ; MOUGHON, S. ; WANG, C. ; SCHUELER-FURMAN, O. ; KUHLMAN, B. ; ROHL, C. A. ; BAKER, D.** *Journal of Molecular Biology,* 2003, vol. 331, 281-300
- **MÉNDEZ, R. ; LEPLAE, R. ; DE MARIA, L. ; WO-DAK, S. J.** *Proteins: Structure, Function, and Bioinformatics,* 2003, vol. 52, 51-67